# EUROPEAN PATENT APPLICATION

(11) **EP 4 707 810 A1**
(43) Date of publication of application: **11.03.2026**
(21) Application number: 24306459.9
(22) Date of filing: 05.09.2024
(51) Int. Cl.: G01N 33/574

(54) **USE OF GLUT1 AS A MARKER FOR HEMATOLOGIC DISORDERS**

(71) Applicant: Metafora Biosystems, 75014 Paris (FR); Assistance Publique - Hôpitaux de Paris, 75012 Paris (FR)
(72) Inventor: PETIT, Vincent, 75014 Paris (FR); VIGNON, Marguerite, 75014 Paris (FR); CHAPUIS, Nicolas, 75014 Paris (FR); BHATNAGAR, Nupur, 75014 Paris (FR); PINSET, Christian, 75014 Paris (FR)
(74) Representative: Icosa

(57) **Abstract**

The present invention relates to the use of the proportion of plasma cells, lymphocytes or blasts expressing GLUT1 among total plasma cells, lymphocytes or blasts in a method for stratifying prognosis of subjects suffering from a hematologic disorder, and in a method for determining whether a subject suffering from a hematologic disorder will achieve or is achieving a response with a modulator of the BCL-2 family proteins. The present invention also relates to a kit for detecting GLUT1, and optionally ASCT2, for implementing one of these methods.

## Description

### FIELD OF INVENTION

The present invention relates to the use of the proportion of plasma cells, lymphocytes or blasts expressing GLUT1 among total plasma cells, lymphocytes or blasts in a method for stratifying prognosis of subjects suffering from a hematologic disorder, and in particular, in subjects suffering from MGUS, SMM or MM. The present invention also relates to the use of the proportion of plasma cells, lymphocytes or blasts expressing GLUT1 among total plasma cells, lymphocytes or blasts in a method for determining whether a subject suffering from a hematologic disorder will achieve or is achieving a response with a modulator of the BCL-2 family proteins, and to a kit for detecting GLUT1, and optionally ASCT2, for implementing one of these methods.

### BACKGROUND OF INVENTION

Hematologic disorders include cancers affecting the blood, bone marrow and lymph nodes, and include, for example, lymphoma, leukemia and multiple myeloma (MM). Multiple myeloma (MM) is a plasma cell malignancy characterized by a clonal proliferation of malignant plasma cells in the bone marrow. Before developing multiple myeloma, subjects may experience two conditions: a monoclonal gammopathy of undetermined significance (MGUS), characterized by low levels of monoclonal protein (M-protein) and abnormal plasma cells in the bone marrow, and smoldering multiple myeloma (SMM), characterized by higher levels of monoclonal protein (M-protein) and of abnormal plasma cells in the bone marrow.

The risk of progression at each step of MM varies greatly depending on the subject, and may be influenced by the disease burden and the underlying cytogenetic type of disease, notably, but other unknown factors might also influence the disease outcome. Overall, the prognostic factors are not fully understood, and more biomarkers are required to refine the prognosis and make the best medical decision.

The Applicants have surprisingly shown that the proportion of plasma cells expressing GLUT1 (*i.e.* GLUT1 positive plasma cells) does not correlate with the disease stage, but correlates with multifactorial risk stratification (prognostic stratification). These data support the use of the proportion of cells expressing GLUT1 as a biomarker for stratifying prognosis in subjects suffering from a hematologic disorder, and in particular, MGUS, SMM or MM.

As the prognosis is also affected by the response to therapy of the subjects, there is also a need to find biomarkers helping to determine the response to a therapy. Interestingly, the Applicants have demonstrated that the proportion of plasma cells expressing GLUT1 also correlates with t(11;14) chromosomal translocation in subjects with MM at diagnosis. As t(11;14) is known as being an efficacy marker of BCL2 inhibitors such as venetoclax, these data support the use of the proportion of cells expressing GLUT1 as a biomarker for evaluating response to therapy, and in particular, to BCL2 modulators.

### SUMMARY

The present invention relates to a method for stratifying prognosis of subjects suffering from a hematologic disorder, comprising a step of determining the proportion of plasma cells, lymphocytes or blasts expressing GLUT1 among total plasma cells, lymphocytes or blasts in a sample obtained from the subjects.

In one embodiment, the hematologic disorder is monoclonal gammopathy of undetermined significance (MGUS), plasmacytoma, smoldering MM (SMM) or multiple myeloma (MM), preferably the hematologic disorder is MGUS, SMM or MM.

In one embodiment, the method is for stratifying, in subjects suffering from MGUS, plasmacytoma or SMM, the risk of progression to MM, and preferably the method is for stratifying, in subjects suffering from MGUS or SMM, the risk of progression to MM.

In one embodiment, the method is for stratifying prognosis, and in particular life expectancy, of subjects suffering from MM.

In one embodiment, said method comprises the steps of:
a) determining the proportion of plasma cells expressing GLUT1 among total plasma cells in samples obtained from the subjects,
b) comparing the proportion of plasma cells expressing GLUT1 determined in step a) with a reference value, and
c) stratifying subjects in risk groups or prognosis groups based on the correlation of the proportion of plasma cells expressing GLUT1 in step a) with the reference value.

In one embodiment, the reference value is either:
- a personalized reference, determined earlier in a same subject, or
- a proportion of plasma cells expressing GLUT1 determined in a reference population.

In one embodiment, a subject is assigned in a high-risk group or poor prognosis group when the proportion of plasma cells expressing GLUT1 measured in step a) is lower than the reference value.

The present invention further relates to a method for determining whether a subject suffering from a hematologic disorder will achieve or is achieving a response with modulator of the BCL-2 family proteins, comprising a step of determining the proportion of plasma cells, lymphocytes or blasts expressing GLUT1 among total plasma cells, lymphocytes or blasts in a sample obtained from the subject.

In one embodiment, said method comprises the steps of:
a) determining the proportion of plasma cells, lymphocytes or blasts expressing GLUT1 among total plasma cells, lymphocytes or blasts in a sample obtained from the subject,
b) comparing the proportion of plasma cells, lymphocytes or blasts expressing GLUT1 determined in step a) with a reference value, and
c) concluding that the subj ect will achieve or is achieving a response with the modulator based on the correlation of the proportion of plasma cells, lymphocytes or blasts expressing GLUT1 in step a) with the reference value.

In one embodiment, it is concluded that the subject will achieve or is achieving a response with the modulator when the proportion of plasma cells, lymphocytes or blasts expressing GLUT1 measured in step a) is higher than the reference value.

In one embodiment, the hematologic disorder is selected from the group comprising or consisting of leukemia, lymphoma, MM, plasmacytoma, MGUS, SMM, myelodysplastic syndromes (MDS) and myeloproliferative neoplasms (MPNs), preferably the hematologic disorder is MM.

In one embodiment, the method is for determining whether a subject suffering from a hematologic disorder will achieve or is achieving a response with an inhibitor of anti-apoptotic BCL-2 family proteins, preferably wherein said inhibitor of anti-apoptotic BCL-2 family proteins is selected from the group comprising or consisting of venetoclax (ABT-199), oblimersen (G3139), obatoclax (GX15-070), navitoclax (ABT-263), pelcitoclax (APG-1252), R-(-)-gossypol acetic acid (AT-101), LP-118, ABT-737, S55746 (BLC201), APG-2575 (lisaftoclax), AZD4320, AZD0466, BM-1197, S44563, DC-B01, A1210477, S63845, DT2216, 753b and WH244, more preferably wherein said inhibitor of anti-apoptotic BCL-2 family proteins is venetoclax.

In one embodiment, the method further comprises a step of measuring the expression of ASCT2 by plasma cells, lymphocytes or blasts in the sample obtained from the subjects.

In one embodiment, GLUT1 and/or ASCT2 expression levels are assessed at the protein level, preferably the measurement of the expression levels of GLUT1 and/or ASCT2 correspond to the detection and/or quantification of GLUT1 and/or ASCT2 on the cell surface, more preferably by detecting and/or quantifying binding of a ligand to GLUT1 or ASCT2, wherein preferably, said ligand is an antibody or is a receptor-binding domain ligand (RBD) comprising a part or the totality of a receptor-binding domain (RBD) derived from the soluble part of a glycoprotein of an enveloped virus.

The present invention further relates to a kit comprising means for detecting GLUT1, and optionally ASCT2, preferably wherein said kit is for implementing a method as described hereinabove.

### DEFINITIONS

In the present invention, the following terms have the following meanings:

"**About**", preceding a figure encompasses plus or minus 10%, or less, of the value of said figure. It is to be understood that the value to which the term "about" refers is itself also specifically, and preferably, disclosed.

"**Conservative substitution**" is one in which an amino acid is substituted by another amino acid that has similar properties, such that one skilled in the art of peptide chemistry would expect the secondary structure and hydropathic nature of the polypeptide to be substantially unchanged. Amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine. Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include histidine, lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. The term "conservative amino acid substitution" may further be defined as an amino acid exchange within one of the following five groups:
I. Small aliphatic, nonpolar or slightly polar residues: Ala, Ser, Thr, Pro, Gly;
II. Polar, negatively charged residues and their amides: Asp, Asn, Glu, Gln;
III. Polar, positively charged residues: His, Arg, Lys;
IV. Large, aliphatic, nonpolar residues: Met, Leu, Ile, Val, Cys;
V. Large, aromatic residues: Phe, Tyr, Trp.

"**Kit**": refers to any manufacture (e.g., a package or a container) comprising at least one reagent for specifically detecting the expression of GLUT1 and/or ASCT2. The kit may be promoted, distributed, or sold as a unit for performing the methods of the present invention. Furthermore, any or all of the kit reagents may be provided within containers that protect them from the external environment, such as in sealed and sterile containers. The kits may also contain a package insert describing the kit and methods for its use.

"**Mammal**" refers to any mammal, including humans, non-human primates, domestic and farm animals, and zoo, sports, or pet animals, such as dogs, cats, cattle, horses, sheep, pigs, goats, rabbits, monkeys, etc. Preferably, the mammal is human.

"**Polypeptide**": refers to a linear polymer of amino acids (preferably at least 50 amino acids) linked together by peptide bonds.

"**Prognosis**" is a medical term for predicting the likelihood or expected development of a disease, including whether the signs and symptoms will improve or worsen (and how quickly) or remain stable over time; the potential for complications and associated health issues; and the likelihood of survival (including life expectancy). A prognosis is made on the basis of the normal course of the diagnosed disease, the individual's physical and mental condition, the available treatments, and additional factors.

"Protein": refers to a functional entity formed of one or more polypeptides, and optionally of non-polypeptides cofactors.

"**Subject**" refers to a mammal, preferably a human. A subject may be a "*patient*", *i.e.*, a warm-blooded animal, more preferably a human, who/which is awaiting the receipt of, or is receiving medical care or was/is/will be the object of a medical procedure, or is monitored for the development of a disease. The term "mammal" refers here to any mammal, including humans, domestic and farm animals, and zoo, sports, or pet animals, such as dogs, cats, cattle, horses, sheep, pigs, goats, rabbits, etc. Preferably, the mammal is a primate, more preferably a human.

"**Therapeutically effective amount**" refers to level or amount of agent that is aimed at, without causing significant negative or adverse side effects to the target, (1) delaying or preventing the onset of an hematologic disorder; (2) slowing down or stopping the progression, aggravation, or deterioration of one or more symptoms of an hematologic disorder; (3) bringing about ameliorations of the symptoms of an hematologic disorder; (4) reducing the severity or incidence of an hematologic disorder; or (5) curing an hematologic disorder. A therapeutically effective amount may be administered prior to the onset of the hematologic disorder, for a prophylactic or preventive action. Alternatively or additionally, the therapeutically effective amount may be administered after initiation of the hematologic disorder, for a therapeutic action.

"**Treatment**" refers to a therapeutic (or curative) treatment, to a prophylactic (or preventive) treatment, or to both a therapeutic (or curative) treatment and a prophylactic (or preventive) treatment, wherein the object is to prevent, reduce, slow down (lessen), or cure one or more of the symptom(s) or manifestation(s) of the diseases as described herein.

"**Variant**" refers to a polypeptide that typically differs from a polypeptide specifically disclosed herein in one or more substitutions, deletions, additions and/or insertions. Such variants may be naturally occurring or may be synthetically generated, for example, by modifying one or more of the above polypeptide sequences and evaluating one or more biological activities of the polypeptide as described herein and/or using any of a number of techniques well known in the art. Modifications may be made in the structure of polypeptides and still obtain a functional molecule that encodes a variant or derivative polypeptide with desirable characteristics.

### DETAILED DESCRIPTION

The present invention relates to the use of GLUT1, and in particular, of the proportion of cells expressing GLUT1, and/or of ASCT2, and in particular of the proportion of cells expressing ASCT2, as biomarkers for hematologic disorders.

As used herein, "GLUT1" refers to glucose transporter 1 (also known as solute carrier family 2, facilitated glucose transporter member 1 (SLC2A1)), a protein that facilitates the transport of glucose through the plasma membrane of mammalian cells. In humans, GLUT1 is encoded by the *SLC2A1* gene. An example of a human GLUT1 is represented by the Uniprot reference P11166 (SEQ ID NO:71). An example of a human *SLC2A1* gene is represented by the NCBI reference Gene ID: 6513.

As used herein, "ASCT2" refers to a glutamine and other neutral amino acid transporter. ASCT2 is used as a receptor for human endogenous retrovirus W (HERV-W), RD114 feline gammaretrovirus, baboon endogenous virus (BaEV), spleen necrosis virus (SNV), simian retrovirus (SRV) and Mason-Pfizer monkey virus (MPMV). In humans, ASCT2 is encoded by the *SLC1A5* gene. An example of a human ASCT2 is represented by the Uniprot reference Q15758.2 (SEQ ID NO: 70). An example of a human *SLC1A5* gene is represented by the reference Gene ID: 6510.

It will be understood by the skilled artisan in the art that the methods described hereinbelow may comprise a step of:
- determining the proportion of plasma cells, lymphocytes or blasts expressing GLUT1 only, or
- determining the proportion of plasma cells, lymphocytes or blasts expressing ASCT2 only, or
- determining both the proportion of plasma cells, lymphocytes or blasts expressing GLUT1 and the proportion of plasma cells, lymphocytes or blasts expressing ASCT2.

In one embodiment of the present invention, the methods described hereinbelow comprise a step of determining the proportion of plasma cells, lymphocytes or blasts expressing GLUT1, and optionally, a step of determining the proportion of plasma cells, lymphocytes or blasts expressing ASCT2.

The present invention relates to a method for stratifying prognosis of subjects suffering from a hematologic disorder, comprising a step of determining i) the proportion of plasma cells, lymphocytes or blasts expressing GLUT1, or ii) the proportion of plasma cells, lymphocytes or blasts expressing ASCT2 or iii) both proportions, among total plasma cells, lymphocytes or blasts in a sample obtained from the subjects.

As used herein, the term "prognosis" refers to predicting the likelihood or expected development of a disease, and thus, may include prediction of disease progression or prediction of survival or life expectancy of subjects.

The method as described herein may comprise the steps of:
a) determining the proportion of plasma cells, lymphocytes or blasts expressing GLUT1 among total plasma cells, lymphocytes or blasts in a sample obtained from the subjects,
b) comparing the proportion of plasma cells, lymphocytes or blasts expressing GLUT1 determined in step a) with a reference value, and
c) stratifying subjects in prognosis groups based on the correlation of the proportion of plasma cells, lymphocytes or blasts expressing GLUT1 determined in step a) with the reference value.

The method as described herein may comprise the steps of:
a) determining the proportion of plasma cells, lymphocytes or blasts expressing ASCT2 among total plasma cells, lymphocytes or blasts in a sample obtained from the subject,
b) comparing the proportion of plasma cells, lymphocytes or blasts determined in step a) with a reference value, and
c) stratifying subjects in prognosis groups based on the correlation of the proportion determined in step a) with the reference value.

The method as described herein may comprise the steps of:
a) determining the proportion of plasma cells, lymphocytes or blasts expressing GLUT1, and the proportion of plasma cells, lymphocytes or blasts expressing ASCT2 among total plasma cells, lymphocytes or blasts in a sample obtained from the subjects,
b) comparing the proportions of plasma cells, lymphocytes or blasts determined in step a) with two reference values for GLUT1 and ASCT2 *(i.e.* one reference value for the proportion of plasma cells, lymphocytes or blasts expressing GLUT1, and one reference value for the proportion of plasma cells, lymphocytes or blasts expressing ASCT2)
c) stratifying subjects in prognosis groups based on the correlation of the proportions determined in step a) with the reference value.

As used herein, prognosis stratification refers to the grading of subjects into incremental levels of prognosis, which may be used to adapt subjects care depending on the identified prognosis.

The stratification of prognosis may be used to make continuous or categorical measurements of the prognosis. In the categorical scenario, the invention may be used to discriminate subjects with a good prognosis (good prognosis subjects) and subjects with a poor prognosis (poor prognosis subjects).

In one embodiment, "good prognosis" refers to greater than average likelihood of survival for a subject suffering from a hematologic disorder as compared to other members of the same gender suffering from the same condition.

In one embodiment, "poor prognosis" refers to a less than average likelihood of survival for a subject suffering from a hematologic disorder as compared to other members of the same gender suffering from the same condition.

The method as described herein may be used to stratify the prognosis for 1, 2, 3, 4, 5 years or more. In particular, the method as described herein may be used to stratify the prognosis for less than 2 years or more than for 7 years.

The poor prognosis group may refer to subjects with a life expectancy of 2 years or less.

The good prognosis group may refer to subjects with a life expectancy of 7 years or more.

According to one embodiment of the present invention, the method as described herein comprises a step of comparing the proportion of plasma cells, lymphocytes or blasts expressing GLUT1, and/or the proportion of plasma cells, lymphocytes or blasts expressing ASCT2, with one or more reference value(s).

As used herein, the term "reference" broadly encompasses any suitable reference of proportion of plasma cells, lymphocytes or blasts expressing GLUT1 or ASCT2 which may be used as a basis for comparison with respect to the measured proportion of plasma cells, lymphocytes or blasts expressing GLUT1 or ASCT2. The reference may be constructed using algorithms and/or other methods of statistical and hierarchical classification. The reference may be stored in a database to provide a stored proportion and the stored proportion is used to determine the difference in the proportion. The database may, for example, be stored on a computer or a server. The reference may also be determined empirically.

A reference proportion can be relative to a number or value derived from population studies, including without limitation, such populations of subjects having similar age range, subjects in the same or similar ethnic group.

When the method as described herein comprises the determination of only the proportion of plasma cells, lymphocytes or blasts expressing GLUT1 or ASCT2, it will be understood by the skilled artisan in the art that the reference value may be the proportion of plasma cells, lymphocytes or blasts expressing GLUT1 or ASCT2 among total plasma cells, lymphocytes or blasts determined in a reference population or a personalized reference of the proportion of plasma cells, lymphocytes or blasts expressing GLUT1 or ASCT2.

When the method as described herein comprises the determination of both the proportion of plasma cells, lymphocytes or blasts expressing GLUT1 and the proportion of plasma cells, lymphocytes or blasts expressing ASCT2, it will be understood by the skilled artisan in the art that:
- the reference value regarding GLUT1 may be the proportion of plasma cells, lymphocytes or blasts expressing GLUT1 among total plasma cells, lymphocytes or blasts determined in a reference population or a personalized reference of the proportion of plasma cells, lymphocytes or blasts expressing GLUT1, and
- the reference value regarding ASCT2 may be the proportion of plasma cells, lymphocytes or blasts expressing ASCT2 among total plasma cells, lymphocytes or blasts determined in a reference population or a personalized reference of the proportion of plasma cells, lymphocytes or blasts expressing ASCT2.

The reference value may be determined in a reference population. The reference population of subjects may comprise at least 40 subjects, at least 100 subjects, at least 500 subjects, at least 1000 subjects, or more.

The reference value may be the proportion of plasma cells, lymphocytes or blasts expressing GLUT1 or ASCT2 among total plasma cells, lymphocytes or blasts determined in a population of subjects suffering a hematologic disorder. The subject may be assigned in a poor prognosis group when:
- the proportion of plasma cells, lymphocytes or blasts expressing GLUT1 determined in step a) is lower than the reference value, when the method comprises the determination of only the proportion of plasma cells, lymphocytes or blasts expressing GLUT1,
- the proportion of plasma cells, lymphocytes or blasts expressing ASCT2 determined in step a) is higher than the reference value, when the method comprises the determination of only the proportion of plasma cells, lymphocytes or blasts expressing ASCT2,
- the proportion of plasma cells, lymphocytes or blasts expressing GLUT1 determined in step a) is lower than the reference value and the proportion of plasma cells, lymphocytes or blasts expressing ASCT2 determined in step a) is higher than the reference value, when the method comprises the determination of the proportion of plasma cells, lymphocytes or blasts expressing GLUT1 and the proportion of plasma cells, lymphocytes or blasts expressing ASCT2.

The reference value may be the proportion of plasma cells determined in a population of subjects suffering from a hematologic disorder and having a poor prognosis. The subject may be assigned in a poor prognosis group when:
- the proportion of plasma cells, lymphocytes or blasts expressing GLUT1 determined in step a) is equal or lower than the reference value, when the method comprises the determination of only the proportion of plasma cells, lymphocytes or blasts expressing GLUT1,
- the proportion of plasma cells, lymphocytes or blasts expressing ASCT2 determined in step a) is equal or higher than the reference value, when the method comprises the determination of only the proportion of plasma cells, lymphocytes or blasts expressing ASCT2,
- the proportion of plasma cells, lymphocytes or blasts expressing GLUT1 determined in step a) is equal or lower than the reference value and the proportion of plasma cells, lymphocytes or blasts expressing ASCT2 determined in step a) is equal or higher than the reference value, when the method comprises the determination of the proportion of plasma cells, lymphocytes or blasts expressing GLUT1 and the proportion of plasma cells, lymphocytes or blasts expressing ASCT2.

The reference value may be the proportion of plasma cells, lymphocytes or blasts determined in a population of subjects suffering from a hematologic disorder and having a good prognosis. The subject may be assigned in a poor prognosis group when:
- the proportion of plasma cells, lymphocytes or blasts expressing GLUT1 determined in step a) is lower than the reference value, when the method comprises the determination of only the proportion of plasma cells, lymphocytes or blasts expressing GLUT1,
- the proportion of plasma cells, lymphocytes or blasts expressing ASCT2 determined in step a) is higher than the reference value when the method comprises the determination of only the proportion of plasma cells, lymphocytes or blasts expressing ASCT2, or
- the proportion of plasma cells, lymphocytes or blasts expressing GLUT1 determined in step a) is lower than the reference value and the proportion of plasma cells, lymphocytes or blasts expressing ASCT2 determined in step a) is higher than the reference value, when the method comprises the determination of the proportion of plasma cells, lymphocytes or blasts expressing GLUT1 and the proportion of plasma cells, lymphocytes or blasts expressing ASCT2.

The information regarding the prognosis of the subjects of the reference population may be deduced from the survival time and/or other information, such as tumor burden (i.e. stage of the disorder) and cytogenetic abnormalities.

The reference proportion of plasma cells, lymphocytes or blasts expressing GLUT1 among total plasma cells, lymphocytes or blasts may be a value comprised from about 10% to about 45%, preferably from about 30% to about 40%. In particular, the reference proportion of plasma cells, lymphocytes or blasts expressing GLUT1 may be one the following values: about 10%, 15%, 20%, 25%, 30%, 35%, 40% or about 45%, preferably about 35%.

The reference value may also be a personalized reference, determined earlier in the same subject. In particular, the reference value may be:
- the proportion of plasma cells, lymphocytes or blasts expressing GLUT1 among total plasma cells determined earlier in the subject, when the method comprises the determination of only the proportion of plasma cells, lymphocytes or blasts expressing GLUT1,
- the proportion of plasma cells, lymphocytes or blasts expressing ASCT2 among total plasma cells determined earlier in the subject, when the method comprises the determination of only the proportion of plasma cells, lymphocytes or blasts expressing ASCT2, or
- the proportion of plasma cells, lymphocytes or blasts expressing GLUT1 among total plasma cells, lymphocytes or blasts and the proportion of plasma cells, lymphocytes or blasts expressing ASCT2 among total plasma cells, lymphocytes or blasts determined earlier in the subject, when the method comprises the determination of the proportion of plasma cells, lymphocytes or blasts expressing GLUT1 and the proportion of plasma cells, lymphocytes or blasts expressing ASCT2.

The subject may be assigned in a poor prognosis group when:
- the proportion of plasma cells, lymphocytes or blasts expressing GLUT1 determined in step a) is lower than the reference value, when the method comprises the determination of only the proportion of plasma cells, lymphocytes or blasts expressing GLUT1,
- the proportion of plasma cells, lymphocytes or blasts expressing ASCT2 determined in step a) is higher than the reference value, when the method comprises the determination of only the proportion of plasma cells, lymphocytes or blasts expressing ASCT2, or
- the proportion of plasma cells, lymphocytes or blasts expressing GLUT1 determined in step a) is lower than the reference value and the proportion of plasma cells, lymphocytes or blasts expressing ASCT2 determined in step a) is higher than the reference value, when the method comprises the determination of the proportion of plasma cells, lymphocytes or blasts expressing GLUT1 and the proportion of plasma cells, lymphocytes or blasts expressing ASCT2.

When the method comprises the determination of only the proportion of plasma cells, lymphocytes or blasts expressing GLUT1, the subject may be assigned in a poor prognosis group when the proportion of plasma cells, lymphocytes or blasts expressing GLUT1 determined in step a) is about 10% or more lower than the second one, such as about 20, 30, 40, 50, 60, 70, 80, 90% or more lower than the second one.

When the method comprises the determination of only the proportion of plasma cells, lymphocytes or blasts expressing ASCT2, the subject may be assigned in a poor prognosis group when the proportion of plasma cells, lymphocytes or blasts expressing ASCT2 determined in step a) is about 10% or more higher than the second one, such as about 20, 30, 40, 50, 60, 70, 80, 90% or more higher than the second one.

When the method comprises the determination of the proportion of plasma cells, lymphocytes or blasts expressing GLUT1 and the proportion of plasma cells, lymphocytes or blasts expressing ASCT2, the subj ect may be assigned in a poor prognosis group when:
- the proportion of plasma cells, lymphocytes or blasts expressing GLUT1 determined in step a) is about 10% or more lower than the second one, such as about 20, 30, 40, 50, 60, 70, 80, 90% or more lower than the second one, and
- the proportion of plasma cells, lymphocytes or blasts expressing ASCT2 determined in step a) is about 10% or more higher than the second one, such as about 20, 30, 40, 50, 60, 70, 80, 90% or more higher than the second one.

The proportion of plasma cells, lymphocytes or blasts expressing GLUT1 or ASCT2 may be determined by measuring the expression levels of GLUT1 and/or ASCT2 as described hereinbelow.

The present invention also relates to a method for treating a subject suffering from a hematologic disorder, comprising the steps of assigning the subject to a prognosis group with a method as described hereinabove, and treating said subject.

The method as described may be used to treat a subj ect with an adapted treatment, depending on the prognosis of the subject.

The method as described herein may comprise the steps of:
a) determining the proportion of plasma cells, lymphocytes or blasts expressing GLUT1 among total plasma cells, lymphocytes or blasts in a sample obtained from the subject,
b) comparing the proportion of plasma cells, lymphocytes or blasts expressing GLUT1 determined in step a) with a reference value,
c) assigning the subject to a prognosis group based on the correlation of the proportion of plasma cells, lymphocytes or blasts expressing GLUT1 determined in step a) with the reference value, and
d) treating the subject with an adapted treatment depending on the prognosis of the subj ect.

The method as described herein may comprise the steps of:
a) determining the proportion of plasma cells, lymphocytes or blasts expressing ASCT2 among total plasma cells, lymphocytes or blasts in a sample obtained from the subject,
b) comparing the proportion of plasma cells, lymphocytes or blasts expressing ASCT2 determined in step a) with a reference value,
c) assigning the subject to a prognosis group based on the correlation of the proportion of plasma cells expressing ASCT2 determined in step a) with the reference value, and
d) treating the subject with an adapted treatment depending on the prognosis of the subj ect.

The method as described herein may comprise the steps of:
a) determining the proportion of plasma cells, lymphocytes or blasts expressing GLUT1, and the proportion of plasma cells, lymphocytes or blasts expressing ASCT2, among total plasma cells in a sample obtained from the subject,
b) comparing the proportions determined in step a) with two reference values for GLUT1 and ASCT2 (i.e. one reference value for the proportion of plasma cells, lymphocytes or blasts expressing GLUT1, and one reference value for the proportion of plasma cells, lymphocytes or blasts expressing ASCT2), and
c) assigning the subject to a prognosis group based on the correlation of the proportions determined in step a) with the reference values, and
d) treating the subject with an adapted treatment depending on the prognosis of the subj ect.

The embodiments described herein for the method to stratify the prognosis of subjects suffering from a hematologic disorder apply *mutatis mutandis* to the method of treatment as described herein.

The embodiments described herein for the method to stratify the prognosis of subjects suffering from a hematologic disorder apply *mutatis mutandis* to the method of treatment as described herein.

Examples of treatments are provided hereinbelow.

The hematologic disorder as described herein may be selected from the group comprising or consisting of leukemia, lymphoma, MM, plasmacytoma, MGUS, SMM, myelodysplastic syndromes (MDS) and myeloproliferative neoplasms (MPNs).

The hematologic disorder as described herein may be selected from the group comprising or consisting of MM, plasmacytoma, MGUS, SMM, acute myeloid leukemia (AML), acute and chronic lymphocytic leukemia (ALL and CLL), myelodysplastic syndromes (MDS), myeloproliferative neoplasms (MPNs) and relapsed/refractory mantle cell lymphoma (MCL).

The hematologic disorder as described herein may be, in particular, MGUS, SMM, plasmacytoma or MM and, more particularly MGUS, SMM or MM.

It is known in the art that the hematologic disorders described herein involve proliferation of different cell types, *i.e.* plasma cells, lymphocytes and blasts. Thus, it will be understood by the skilled artisan in art that the method as described herein comprises a step of determining, in the cell type involved in the hematologic disorder, i) the proportion of cells expressing GLUT1, or ii) the proportion of cells expressing ASCT2 or iii) both proportions, among total cells from the same cell type.

Leukemia, MDS, and MPNs involve abnormal proliferation of blasts in the blood and lymph nodes. As used herein, the term "blasts" or "blast cells" refers to immature cells, which give rise to all kinds of different specialized cells. These cells are also referred as "precursor cells" or "stem cells".

Thus, the present invention relates to a method for stratifying prognosis of subjects suffering from leukemia, such as AML, ALL or CLL, MDS or MPNs, comprising a step of determining i) the proportion of blast cells expressing GLUT1, or ii) the proportion of blast cells expressing ASCT2 or iii) both proportions, among total blast cells in a sample obtained from the subject.

MGUS, plasmacytoma, SMM and MM involves abnormal proliferation of plasma cells. As used herein, the term "plasma cells" refers to differentiated B-lymphocyte white blood cells capable of secreting immunoglobulin or antibodies. These cells are the main cells responsible for humoral immunity. These cells are also referred as "plasmocytes".

Thus, the present invention relates to a method for stratifying prognosis of subjects suffering from MGUS, plasmacytoma, SMM or MM, comprising a step of determining the proportion of plasma cells expressing GLUT1, or ii) the proportion of plasma cells expressing ASCT2 or iii) both proportions, among total plasma cells in a sample obtained from the subject.

Lymphoma involves abnormal proliferation of lymphocytes. As used herein, the term "lymphocytes" refers to white blood cells formed in the lymphoid tissue. Lymphocytes include T cells, B cells and innate lymphoid cells, these latter including natural killer cells (NK cells).

Thus, the present invention relates to a method for stratifying risk or prognosis of subjects suffering from lymphoma, such as MCL, comprising a step of determining i) the proportion of lymphocytes expressing GLUT1, or ii) the proportion of lymphocytes expressing ASCT2 or iii) both proportions, among total lymphocytes in a sample obtained from the subject.

The method as described herein may be used to stratify, in subjects suffering from MGUS, plasmacytoma or SMM, the risk of progression to MM.

Thus, the present invention also relates to a method for stratifying, in subjects suffering from MGUS, plasmacytoma or SMM, the risk of progression to MM, comprising a step of determining i) the proportion of plasma cells expressing GLUT1, ii) the proportion of plasma cells expressing ASCT2, or iii) both proportions, among total plasma cells in a sample obtained from the subjects.

More particularly, the method as described herein may be used to stratify, in subjects suffering from MGUS or SMM, the risk of progression to MM.

Thus, the present invention also relates to a method for stratifying, in subjects suffering from MGUS or SMM, the risk of progression to MM, comprising a step of determining i) the proportion of plasma cells expressing GLUT1, ii) the proportion of plasma cells expressing ASCT2, or iii) both proportions, among total plasma cells in a sample obtained from the subjects.

The method as described may be used to stratify the risk of progression from MGUS, plasmacytoma or SMM to MM in subjects, and to optimize both the treatment and the monitoring schedule of the subjects. In particular, the method as described herein may allow to treat subjects with a high-risk of progression to MM before the development of severe symptoms or complications.

The method as described herein may comprise the steps of:
a) determining the proportion of plasma cells expressing GLUT1 among total plasma cells in a sample obtained from the subjects,
b) comparing the proportion of plasma cells expressing GLUT1 determined in step a) with a reference value, and
c) stratifying subjects in risk groups based on the correlation of the proportion of plasma cells expressing GLUT1 determined in step a) with the reference value.

The method as described herein may comprise the steps of:
a) determining the proportion of plasma cells expressing ASCT2, among total plasma cells in a sample obtained from the subjects,
b) comparing the proportion determined in step a) with a reference value, and
c) stratifying subjects in risk groups based on the correlation of the proportion determined in step a) with the reference value.

The method as described herein may comprises the steps of:
a) determining the proportion of plasma cells expressing GLUT1, and the proportion of plasma cells expressing ASCT2, among total plasma cells in a sample obtained from the subjects,
b) comparing the proportions determined in step a) with two reference values for GLUT1 and ASCT2 (*i.e.* one reference value for the proportion of plasma cells expressing GLUT1, and one reference value for the proportion of plasma cells expressing ASCT2), and
c) stratifying subjects in risk groups based on the correlation of the proportions determined in step a) with the reference values.

As used herein, MM relates to a symptomatic condition characterized by a clonal proliferation of malignant plasma cells in the bone marrow. MM may develop from MGUS that progresses to SMM.

As used herein, MGUS relates to a condition characterized by low levels of monoclonal protein (M-protein) and abnormal plasma cells in the bone marrow. There are three different subtypes of MGUS according to the type of monoclonal protein: non-IgM MGUS, IgM MGUS and light chain MGUS.

As used herein, SMM relates to a condition, which is an intermediate stage between MGUS and MM, characterized by higher levels of monoclonal protein (M-protein) and abnormal plasma cells in the bone marrow.

As used herein, plasmacytoma relates to a monoclonal proliferation of neoplastic plasma cells at only one anatomic site, which may eventually evolve into MM.

MGUS, SMM, plasmacytoma and MM may be diagnosed by routine laboratory tests including tests to assess blood cells, kidney function, or monoclonal proteins; bone marrow tests and imaging.

MGUS, SMM, plasmacytoma and MM may be diagnosed according to the International Myeloma Working Group (IMGW) diagnostic criteria (Rajkumar, Am J Hematol. 2024 Jun 28).

IMGW diagnostic criteria for diagnosing MGUS, plasmacytoma, SMM or MM are presented in Table 1.

**Table 1**

| **Plasma-cell disorder** | **Definition** | | |
|---|---|---|---|
| Non-IgM MGUS | All 3 criteria must be met: | | |
| | | • Serum monoclonal protein (non-IgM type) <3 gm/dl | |
| | | • Clonal bone marrow plasma cells <10% | |
| | | • Absence of end-organ damage such as hypercalcemia, renal insufficiency, anemia, and bone lesions (CRAB) that can be attributed to the plasma cell proliferative disorder | |
| SMM | Both criteria must be met: | | |
| | | • Serum monoclonal protein (IgG or IgA) ≥30 g/L or urinary monoclonal protein ≥500 mg per 24 h and/or clonal bone marrow plasma cells 10-60% | |
| | | • Absence of myeloma defining events or amyloidosis | |
| MM | Both criteria must be met: | | |
| | | • Clonal bone marrow plasma cells ≥10% or biopsy-proven bony or extramedullary plasmacytoma | |
| | | • Any one or more of the following myeloma defining events: | |
| | | - Evidence of end organ damage that can be attributed to the underlying plasma cell proliferative disorder, specifically: | |
| | | | ∘ Hypercalcaemia: serum calcium >0.25 mmol/L (>1 mg/dl) higher than the upper limit of normal or >2.75 mmol/L (>11 mg/dl) |
| | | | ∘ Renal insufficiency: creatinine clearance <40 mL per minute or serum creatinine >177 µmol/L (>2 mg/dl) |
| | | | ∘ Anaemia: hemoglobin value of >2 g/dl below the lower limit of normal, or a hemoglobin value <10 g/dl |
| | | | ∘ Bone lesions: one or more osteolytic lesions on skeletal radiography, computed tomography (CT), or positron emission tomography-CT (PET-CT). |
| | | - Clonal bone marrow plasma cell percentage ≥ 60% | |
| | | - Involved: uninvolved serum free light chain (FLC) ratio ≥100 (involved free light chain level must be ≥100 mg/L) | |
| | | - >1 focal lesions on magnetic resonance imaging (MRI) studies (at least 5 mm in size) | |
| IgM MGUS | All 3 criteria must be met: | | |
| | | | • Serum IgM monoclonal protein <3 gm/dl |
| | | | • Bone marrow lymphoplasmacytic infiltration <10% |
| | | | • No evidence of anemia, constitutional symptoms, hyperviscosity, lymphadenopathy, or hepatosplenomegaly that can be attributed to the underlying lymphoproliferative disorder. |
| Light chain MGUS | All criteria muste be met: | | |
| | | | • Abnormal FLC ratio (<0.26 or >1.65) |
| | | | • Increased level of the appropriate involved light chain (increased κ FLC in subjectss with ratio >1.65 and increased λ FLC in subjectss with ratio <0.26) |
| | | | • No immunoglobulin heavy chain expression on immunofixation |
| | | | • Absence of end-organ damage that can be attributed to the plasma cell proliferative disorder |
| | | | • Clonal bone marrow plasma cells <10% |
| | | | • Urinary monoclonal protein <500mg/24h |
| Solitary plasmacytoma | All 4 criteria must be met | | |
| | | | • Biopsy proven solitary lesion of bone or soft tissue with evidence of clonal plasma cells |
| | | | • Normal bone marrow with no evidence of clonal plasma cells |
| | | | • Normal skeletal survey and MRI (or CT) of spine and pelvis (except for the primary solitary lesion) |
| | | | • Absence of end-organ damage such as hypercalcemia, renal insufficiency, anemia, or bone lesions(CRAB) that can be attributed to a lympho-plasma cell proliferative disorder |
| Solitary plasmacytoma with minimal marrow involvement | All 4 criteria must be met | | |
| | | | • Biopsy proven solitary lesion of bone or soft tissue with evidence of clonal plasma cells |
| | | | • Clonal bone marrow plasma cells <10% |
| | | | • Normal skeletal survey and MRI (or CT) of spine and pelvis (except for the primary solitary lesion) |
| | | | • Absence of end-organ damage such as hypercalcemia, renal insufficiency, anemia, or bone lesions(CRAB) that can be attributed to a lympho-plasma cell proliferative disorder |

As used herein, the expression "predicting the risk of progression" encompasses making a prediction of the probability, odds, or likelihood of a conversion from one disease state (e.g. MGUS, plasmacytoma or MM) to another (e.g. MM).

The determination of the risk of progressing from MGUS, plasmacytoma or SMM to MM may be used to make continuous or categorical measurements of the risk of conversion, thus defining a spectrum of risks of conversion of subjects. In the categorical scenario, the invention may be used to discriminate between:
- subjects with a low or intermediate risk of conversion (favorable-risk and intermediate-risk subjects), and
- subjects with a high risk of conversion (high-risk subjects).

According to one embodiment of the invention, the method as described herein comprises a step of comparing the proportion of plasma cells expressing GLUT1, and/or the proportion of plasma cells expressing ASCT2, with one or more reference value(s).

The reference value may be determined as described hereinabove.

The reference value may be determined in a reference population. The reference population of subjects may comprise at least 40 subjects, at least 100 subjects, at least 500 subjects, least 1000 subjects, or more.

The reference value may be the proportion of plasma cells expressing GLUT1 or ASCT2 among total plasma cells determined in a population of subjects suffering from MGUS, plasmacytoma, SMM or MM. The subject may be assigned with a high risk of progressing from MGUS, plasmacytoma or SMM to MM when:
- the proportion of plasma cells expressing GLUT1 determined in step a) is lower than the reference value, when the method comprises the determination of only the proportion of plasma cells expressing GLUT1,
- the proportion of plasma cells expressing ASCT2 determined in step a) is higher than the reference value, when the method comprises the determination of only the proportion of plasma cells expressing ASCT2,
- the proportion of plasma cells expressing GLUT1 determined in step a) is lower than the reference value and the proportion of plasma cells expressing ASCT2 determined in step a) is higher than the reference value, when the method comprises the determination of the proportion of plasma cells expressing GLUT1 and the proportion of plasma cells expressing ASCT2.

The reference value may be the proportion of plasma cells expressing GLUT1 or ASCT2 among total plasma cells determined in a population of subjects suffering from MGUS, SMM or MM. The subject may be assigned with a high risk of progressing from MGUS or SMM to MM when:
- the proportion of plasma cells expressing GLUT1 determined in step a) is lower than the reference value, when the method comprises the determination of only the proportion of plasma cells expressing GLUT1,
- the proportion of plasma cells expressing ASCT2 determined in step a) is higher than the reference value, when the method comprises the determination of only the proportion of plasma cells expressing ASCT2,
- the proportion of plasma cells expressing GLUT1 determined in step a) is lower than the reference value and the proportion of plasma cells expressing ASCT2 determined in step a) is higher than the reference value, when the method comprises the determination of the proportion of plasma cells expressing GLUT1 and the proportion of plasma cells expressing ASCT2.

The reference value may be the proportion of plasma cells determined in a population of subjects suffering from MGUS or SMM and being high-risk of progressing from MGUS or SMM to MM. The subject may be assigned with a high risk of progression from MGUS or SMM to MM when:
- the proportion of plasma cells expressing GLUT1 determined in step a) is equal or lower than the reference value, when the method comprises the determination of only the proportion of plasma cells expressing GLUT1,
- the proportion of plasma cells expressing ASCT2 determined in step a) is equal or higher than the reference value, when the method comprises the determination of only the proportion of plasma cells expressing ASCT2,
- the proportion of plasma cells expressing GLUT1 determined in step a) is equal or lower than the reference value and the proportion of plasma cells expressing ASCT2 determined in step a) is equal or higher than the reference value, when the method comprises the determination of the proportion of plasma cells expressing GLUT1 and the proportion of plasma cells expressing ASCT2.

The reference value may be the proportion of plasma cells determined in a population of subjects suffering from MGUS or SMM and being low-risk of progressing from MGUS or SMM to MM. The subject may be assigned with a high risk of progression from MGUS or SMM to MM when:
- the proportion of plasma cells expressing GLUT1 determined in step a) is lower than the reference value, when the method comprises the determination of only the proportion of plasma cells expressing GLUT1,
- the proportion of plasma cells expressing ASCT2 determined in step a) is higher than the reference value when the method comprises the determination of only the proportion of plasma cells expressing ASCT2, or
- the proportion of plasma cells expressing GLUT1 determined in step a) is lower than the reference value and the proportion of plasma cells expressing ASCT2 determined in step a) is higher than the reference value, when the method comprises the determination of the proportion of plasma cells expressing GLUT1 and the proportion of plasma cells expressing ASCT2.

The reference proportion of plasma cells expressing GLUT1 among total plasma cells may be a value comprised from about 10% to about 45%, preferably from about 30% to about 40%. In particular, the reference proportion of plasma cells expressing GLUT1 may be one of the following values: about 10%, 15%, 20%, 25%, 30%, 35%, 40% or about 45%, preferably about 35%.

The reference value may also be a personalized reference, determined earlier in the same subject. In particular, the reference value may be:
- the proportion of plasma cells expressing GLUT1 among total plasma cells determined earlier in the subject, when the method comprises the determination of only the proportion of plasma cells expressing GLUT1,
- the proportion of plasma cells expressing ASCT2 among total plasma cells determined earlier in the subject, when the method comprises the determination of only the proportion of plasma cells expressing ASCT2, or
- the proportion of plasma cells expressing GLUT1 among total plasma cells and the proportion of plasma cells expressing ASCT2 among total plasma cells determined earlier in the subject, when the method comprises the determination of the proportion of plasma cells expressing GLUT1 and the proportion of plasma cells expressing ASCT2.

The subject may be assigned with a high risk of progression from MGUS or SMM to MM when:
- the proportion of plasma cells expressing GLUT1 determined in step a) is lower than the reference value, when the method comprises the determination of only the proportion of plasma cells expressing GLUT1,
- the proportion of plasma cells expressing ASCT2 determined in step a) is higher than the reference value, when the method comprises the determination of only the proportion of plasma cells expressing ASCT2, or
- the proportion of plasma cells expressing GLUT1 determined in step a) is lower than the reference value and the proportion of plasma cells expressing ASCT2 determined in step a) is higher than the reference value, when the method comprises the determination of the proportion of plasma cells expressing GLUT1 and the proportion of plasma cells expressing ASCT2.

When the method comprises the determination of only the proportion of plasma cells expressing GLUT1, the subject may be assigned with a high risk of progression from MGUS or SMM to MM when the proportion of plasma cells expressing GLUT1 determined in step a) is about 10% or more lower than the second one, such as about 20, 30, 40, 50, 60, 70, 80, 90% or more lower than the second one.

When the method comprises the determination of only the proportion of plasma cells expressing ASCT2, the subject may be assigned with a high risk of progression from MGUS or SMM to MM when the proportion of plasma cells expressing ASCT2 determined in step a) is about 10% or more higher than the second one, such as about 20, 30, 40, 50, 60, 70, 80, 90% or more higher than the second one.

When the method comprises the determination of the proportion of plasma cells expressing GLUT1 and the proportion of plasma cells expressing ASCT2, the subject may be assigned with a high risk of progression from MGUS or SMM to MM when:
- the proportion of plasma cells expressing GLUT1 determined in step a) is about 10% or more lower than the second one, such as about 20, 30, 40, 50, 60, 70, 80, 90% or more lower than the second one, and
- the proportion of plasma cells expressing ASCT2 determined in step a) is about 10% or more higher than the second one, such as about 20, 30, 40, 50, 60, 70, 80, 90% or more higher than the second one.

The proportion of plasma cells expressing GLUT1 or ASCT2 may be determined by measuring the expression levels of GLUT1 and/or ASCT2 as described hereinbelow.

The present invention also relates to a method for treating a subject suffering from MGUS, plasmacytoma or SMM, comprising the steps of determining the risk of progression to MM in the subject with a method as described hereinabove, and treating said subject.

The present invention also relates to a method for treating a subject suffering from MGUS or SMM, comprising the steps of determining the risk of progression to MM in the subject with a method as described hereinabove, and treating said subject.

The method as described herein may be used to treat a subject with an adapted treatment, depending on the risk of progression to MM of the subject.

The method as described herein may comprise the steps of:
a) determining the proportion of plasma cells expressing GLUT1 among total plasma cells in a sample obtained from the subject,
b) comparing the proportion of plasma cells expressing GLUT1 determined in step a) with a reference value,
c) assigning the subject to a risk group based on the correlation of the proportion of plasma cells expressing GLUT1 determined in step a) with the reference value, and
d) treating the subject with an adapted treatment depending on the risk of progression of the subject.

The method as described herein may comprise the steps of:
a) determining the proportion of plasma cells expressing ASCT2 among total plasma cells in a sample obtained from the subject,
b) comparing the proportion of plasma cells expressing ASCT2 determined in step a) with a reference value,
c) assigning the subject to a risk group based on the correlation of the proportion of plasma cells expressing ASCT2 determined in step a) with the reference value, and
d) treating the subj ect with an adapted treatment depending on the risk of progression of the subject.

The method as described herein may comprise the steps of:
a) determining the proportion of plasma cells expressing GLUT1, and the proportion of plasma cells expressing ASCT2, among total plasma cells in a sample obtained from the subject,
b) comparing the proportions determined in step a) with two reference values for GLUT1 and ASCT2 *(i.e.* one reference value for the proportion of plasma cells expressing GLUT1, and one reference value for the proportion of plasma cells expressing ASCT2), and
c) assigning the subject to a risk group based on the correlation of the proportions determined in step a) with the reference values, and
d) treating the subj ect with an adapted treatment depending on the risk of progression of the subject.

Examples of treatments are provided hereinbelow.

When the subject suffers from MGUS and is assigned to a high risk of progression, said subject may be treated with lenalidomide or lenalidomide plus dexamethasone.

When the subject suffers from MGUS and is assigned to a low risk of progression, said subject may be under active surveillance.

As used herein, "active surveillance" (or "watchful waiting") means closely monitoring a subject's condition without giving any treatment until symptoms appear or change.

The embodiments described herein for the method to determine the risk of progression from MGUS, plasmacytoma or SMM to MM in subject apply *mutatis mutandis* to the method of treatment as described herein.

The method as described herein may be used to stratify prognosis, and in particular life expectancy, of subjects suffering from MM.

Thus, the present invention also relates to a method for stratifying prognosis of subjects suffering from MM, comprising a step of determining i) the proportion of plasma cells expressing GLUT1, ii) the proportion of plasma cells expressing ASCT2, or iii) both proportions, among total plasma cells in a sample obtained from the subject.

The method as described may be used to determine the prognosis, and in particular the life expectancy, of a subject, and to optimize both the treatment and the monitoring schedule of the subject.

Crab criteria and genetic anomalies used in IMGW consensus for risk stratification and associated median overall survival are presented in Table 2 below (Chng et al., Leukemia. 2014 Feb;28(2):269-77).

**Table 2**

| | Parameters | | Median overall survival | % subjects |
|---|---|---|---|---|
| High-risk | | • International stating system (ISS) : II/III | 2 years | 20% |
| | | • t(4;14)a or 17p13 del | | |
| Intermediate risk | | • Others | 7 years | 60% |
| Favorable risk | | • ISS I/II | > 10 years | 20% |
| | | • absence of t(4;14), 17p13 del and +1q21 | | |
| | | • age < 55 years | | |

The stratification of prognosis may be used to make continuous or categorical measurements of the prognosis. In the categorical scenario, the invention may be used to discriminate: subjects with a good prognosis (good prognosis subjects) and subjects with a poor prognosis (poor prognosis subjects).

The poor prognosis group may refer to subjects with a life expectancy of 2 years or less.

The good prognosis group may refer to subjects with a life expectancy of 7 years or more.

Examples of factors influencing the prognosis include, for example, tumor burden (i.e. stage of the disorder) and cytogenetic abnormalities.

Tumor burden may be assessed by several classification systems, such as, for example, Durie-Salmon Staging (DSS) and the International Staging System (ISS). The presence of cytogenetic abnormalities may be assessed by detecting cytogenetic abnormalities such as del(17p), gain(1q), or del(1p).

According to one embodiment of the invention, the method as described herein comprises a step of comparing the proportion of plasma cells expressing GLUT1, and/or the proportion of plasma cells expressing ASCT2, with one or more reference value(s).

The reference value may be determined as described hereinabove.

The reference value may be determined in a reference population. The reference population of subjects may comprise at least 40 subjects, at least 100 subjects, at least 500 subjects, least 1000 subjects, or more.

The reference value may be the proportion of plasma cells expressing GLUT1 or ASCT2 among total plasma cells determined in a population of subjects suffering from MM or plasmacytoma. The subject may be assigned in a poor prognosis group when:
- the proportion of plasma cells expressing GLUT1 determined in step a) is lower than the reference value, when the method comprises the determination of only the proportion of plasma cells expressing GLUT1,
- the proportion of plasma cells expressing ASCT2 determined in step a) is higher than the reference value, when the method comprises the determination of only the proportion of plasma cells expressing ASCT2,
- the proportion of plasma cells expressing GLUT1 determined in step a) is lower than the reference value and the proportion of plasma cells expressing ASCT2 determined in step a) is higher than the reference value, when the method comprises the determination of the proportion of plasma cells expressing GLUT1 and the proportion of plasma cells expressing ASCT2.

The reference value may be the proportion of plasma cells expressing GLUT1 or ASCT2 among total plasma cells determined in a population of subjects suffering from MM. The subject may be assigned in a poor prognosis group when:
- the proportion of plasma cells expressing GLUT1 determined in step a) is lower than the reference value, when the method comprises the determination of only the proportion of plasma cells expressing GLUT1,
- the proportion of plasma cells expressing ASCT2 determined in step a) is higher than the reference value, when the method comprises the determination of only the proportion of plasma cells expressing ASCT2,
- the proportion of plasma cells expressing GLUT1 determined in step a) is lower than the reference value and the proportion of plasma cells expressing ASCT2 determined in step a) is higher than the reference value, when the method comprises the determination of the proportion of plasma cells expressing GLUT1 and the proportion of plasma cells expressing ASCT2.

The reference value may be the proportion of plasma cells determined in a population of subjects suffering from MM and having a poor prognosis. The subject may be assigned in a poor prognosis group when:
- the proportion of plasma cells expressing GLUT1 determined in step a) is equal or lower than the reference value, when the method comprises the determination of only the proportion of plasma cells expressing GLUT1,
- the proportion of plasma cells expressing ASCT2 determined in step a) is equal or higher than the reference value, when the method comprises the determination of only the proportion of plasma cells expressing ASCT2,
- the proportion of plasma cells expressing GLUT1 determined in step a) is equal or lower than the reference value and the proportion of plasma cells expressing ASCT2 determined in step a) is equal or higher than the reference value, when the method comprises the determination of the proportion of plasma cells expressing GLUT1 and the proportion of plasma cells expressing ASCT2.

The reference value may be the proportion of plasma cells determined in a population of subjects suffering from MM and having a good prognosis. The subject may be assigned in a poor prognosis group when:
- the proportion of plasma cells expressing GLUT1 determined in step a) is lower than the reference value, when the method comprises the determination of only the proportion of plasma cells expressing GLUT1,
- the proportion of plasma cells expressing ASCT2 determined in step a) is higher than the reference value when the method comprises the determination of only the proportion of plasma cells expressing ASCT2, or
- the proportion of plasma cells expressing GLUT1 determined in step a) is lower than the reference value and the proportion of plasma cells expressing ASCT2 determined in step a) is higher than the reference value, when the method comprises the determination of the proportion of plasma cells expressing GLUT1 and the proportion of plasma cells expressing ASCT2.

The reference proportion of plasma cells expressing GLUT1 among total plasma cells may be a value comprised from about 10% to about 45%, preferably from about 30% to about 40%. In particular, the reference proportion of plasma cells expressing GLUT1 may be one the following values: about 10%, 15%, 20%, 25%, 30%, 35%, 40% or about 45%, preferably about 35%.

The reference value may also be a personalized reference, determined earlier in the same subject. In particular, the reference value may be:
- the proportion of plasma cells expressing GLUT1 among total plasma cells determined earlier in the subject, when the method comprises the determination of only the proportion of plasma cells expressing GLUT1,
- the proportion of plasma cells expressing ASCT2 among total plasma cells determined earlier in the subject, when the method comprises the determination of only the proportion of plasma cells expressing ASCT2, or
- the proportion of plasma cells expressing GLUT1 among total plasma cells and the proportion of plasma cells expressing ASCT2 among total plasma cells determined earlier in the subject, when the method comprises the determination of the proportion of plasma cells expressing GLUT1 and the proportion of plasma cells expressing ASCT2.

The subject may be assigned in a poor prognosis group when:
- the proportion of plasma cells expressing GLUT1 determined in step a) is lower than the reference value, when the method comprises the determination of only the proportion of plasma cells expressing GLUT1,
- the proportion of plasma cells expressing ASCT2 determined in step a) is higher than the reference value, when the method comprises the determination of only the proportion of plasma cells expressing ASCT2, or
- the proportion of plasma cells expressing GLUT1 determined in step a) is lower than the reference value and the proportion of plasma cells expressing ASCT2 determined in step a) is higher than the reference value, when the method comprises the determination of the proportion of plasma cells expressing GLUT1 and the proportion of plasma cells expressing ASCT2.

When the method comprises the determination of only the proportion of plasma cells expressing GLUT1, the subject may be assigned in a poor prognosis group when the proportion of plasma cells expressing GLUT1 determined in step a) is about 10% or more lower than the second one, such as about 20, 30, 40, 50, 60, 70, 80, 90% or more lower than the second one.

When the method comprises the determination of only the proportion of plasma cells expressing ASCT2, the subject may be assigned in a poor prognosis group when the proportion of plasma cells expressing ASCT2 determined in step a) is about 10% or more higher than the second one, such as about 20, 30, 40, 50, 60, 70, 80, 90% or more higher than the second one.

When the method comprises the determination of the proportion of plasma cells expressing GLUT1 and the proportion of plasma cells expressing ASCT2, the subject may be assigned in a poor prognosis group when:
- the proportion of plasma cells expressing GLUT1 determined in step a) is about 10% or more lower than the second one, such as about 20, 30, 40, 50, 60, 70, 80, 90% or more lower than the second one, and
- the proportion of plasma cells expressing ASCT2 determined in step a) is about 10% or more higher than the second one, such as about 20, 30, 40, 50, 60, 70, 80, 90% or more higher than the second one.

The proportion of plasma cells expressing GLUT1 or ASCT2 may be determined by measuring the expression levels of GLUT1 and/or ASCT2 as described hereinbelow.

The present invention also relates to a method for treating a subject suffering from MM, comprising the steps of assigning the subject to a prognosis group with a method as described hereinabove, and treating said subject.

The method as described herein may be used to treat a subject with an adapted treatment, depending on the identified prognosis.

The method as described herein may comprise the steps of:
a) determining the proportion of plasma cells expressing GLUT1 among total plasma cells in a sample obtained from the subject,
b) comparing the proportion of plasma cells expressing GLUT1 determined in step a) with a reference value,
c) assigning the subject to a prognosis group based on the correlation of the proportion of plasma cells expressing GLUT1 determined in step a) with the reference value, and
d) treating the subject with an adapted treatment depending on the identified prognosis.

The method as described herein may comprise the steps of:
a) determining the proportion of plasma cells expressing ASCT2 among total plasma cells in a sample obtained from the subject,
b) comparing the proportion of plasma cells expressing ASCT2 determined in step a) with a reference value,
c) assigning the subject to a prognosis group based on the correlation of the proportion of plasma cells expressing ASCT2 determined in step a) with the reference value, and
d) treating the subject with an adapted treatment depending on the identified prognosis.

The method as described herein may comprise the steps of:
a) determining the proportion of plasma cells expressing GLUT1, and the proportion of plasma cells expressing ASCT2, among total plasma cells in a sample obtained from the subject,
b) comparing the proportions determined in step a) with two reference values for GLUT1 and ASCT2 (*i.e.* one reference value for the proportion of plasma cells expressing GLUT1, and one reference value for the proportion of plasma cells expressing ASCT2), and
c) assigning the subject to a prognosis group based on the correlation of the proportions determined in step a) with the reference values, and
d) treating the subject with an adapted treatment depending on the identified prognosis.

Examples of treatments include, without limitation, Bortezomib-thalidomide-dexamethasone (VTd), Bortezomib-cyclophosphamide-dexamethasone (VCd or CyBord), Bortezomib-lenalidomide-dexamethasone (VRd), Carfilzomib-cyclophosphamide-dexamethasone (KCd), Carfilzomib-lenalidomide-dexamethasone (KRd), Carfilzomib-pomalidomide-dexamethasone (KPd), Daratumumab-lenalidomide-dexamethasone (DRd), Daratumumab-bortezomib-dexamethasone (DVd), Daratumumab-pomalidomide-dexamethasone (DPd), Daratumumab-carfilzomib-dexamethasone (DKd), Ixazomib-lenalidomide-dexamethasone (IRd), Elotuzumab-pomalidomide-dexamethasone (EPd), Isatuximab-pomalidomide-dexamethasone (Isa-Pd), and Isatuximab-carfilzomib-dexamethasoneb (Isa-Kd).

Examples of treatments also include anti-CD38 antibodies such as daratumumab, belantamab mafodotin, anti-BCMA (B-cell maturation antigen) antibodies, and anti-slam7 antibodies such as elotuzumab.

When the subject suffers from MM and is assigned to a bad prognosis, the step d) may comprise treating the subject with carfilzomib, lenalidomide, pomalidomide, double autografting, and/or anti-CD38 antibodies.

When the subject suffers from MM and is assigned to a good prognosis, the step d) may comprise treating the subject with bortezomib.

The embodiments described herein for the method for stratifying prognosis of subjects with MM apply *mutatis mutandis* to the method of treatment as described herein.

The present invention relates to a method for determining whether a subject suffering from a hematologic disorder will achieve or is achieving a response with a modulator of the BCL-2 family proteins, comprising a step of determining i) the proportion of plasma cells, lymphocytes or blasts expressing GLUT 1, or ii) the proportion of plasma cells, lymphocytes or blasts expressing ASCT2 or iii) both proportions, among total plasma cells, lymphocytes or blasts in a sample obtained from the subject.

As used herein, a modulator of BCL-2 family proteins refers to an agent capable of modifying the expression and/or the function, such as the pro- or the anti-apoptotic function, of the proteins of the BCL-2 family.

The modulator of BCL-2 family proteins may be an inhibitor of anti-apoptotic BCL-2 family proteins.

Thus, in one embodiment of the invention, the present invention relates to a method for determining whether a subject suffering from a hematologic disorder will achieve or is achieving a response with an inhibitor of the anti-apoptotic BCL-2 family proteins, comprising a step of i) determining the proportion of plasma cells, lymphocytes or blasts expressing GLUT1, or ii) the proportion of plasma cells, lymphocytes or blasts expressing ASCT2 or iii) both proportions, among total plasma cells, lymphocytes or blasts in a sample obtained from the subject.

The modulator of BCL-2 family proteins may be an agent modulating phosphorylation or stability of the BCL-2 family proteins.

Thus, in one embodiment of the invention, the present invention relates to a method for determining whether a subject suffering from a hematologic disorder will achieve or is achieving a response with an agent modulating phosphorylation or stability of the BCL-2 family proteins, comprising a step of determining i) the proportion of plasma cells, lymphocytes or blasts expressing GLUT1, or ii) the proportion of plasma cells, lymphocytes or blasts expressing ASCT2 or iii) both proportions, among total plasma cells, lymphocytes or blasts in a sample obtained from the subject.

The method as described herein may be used to determine the responsiveness of a subject suffering from a hematologic disorder to a modulator as described herein, and to optimize the treatment of the subject.

The method as described herein may comprise the steps of:
a) determining the proportion of plasma cells, lymphocytes or blasts expressing GLUT1 among total plasma cells, lymphocytes or blasts in a sample obtained from the subject,
b) comparing the proportion of plasma cells, lymphocytes or blasts expressing GLUT1 determined in step a) with a reference value, and
c) concluding that the subject will achieve or is achieving a response with a modulator as described herein based on the correlation of the proportion of plasma cells, lymphocytes or blasts expressing GLUT1 determined in step a) with the reference value.

The method as described herein may comprise the steps of:
a) determining the proportion of plasma cells, lymphocytes or blasts expressing ASCT2 among total plasma cells, lymphocytes or blasts in a sample obtained from the subject,
b) comparing the proportion of plasma cells, lymphocytes or blasts determined in step a) with a reference value, and
c) concluding that the subject will achieve or is achieving a response with a modulator as described herein based on the correlation of the proportion determined in step a) with the reference value.

The method as described herein may comprise the steps of:
d) determining the proportion of plasma cells, lymphocytes or blasts expressing GLUT1, and the proportion of plasma cells, lymphocytes or blasts expressing ASCT2 among total plasma cells, lymphocytes or blasts in a sample obtained from the subject,
e) comparing the proportions of plasma cells, lymphocytes or blasts determined in step a) with two reference values for GLUT1 and ASCT2 *(i.e.* one reference value for the proportion of plasma cells, lymphocytes or blasts expressing GLUT1, and one reference value for the proportion of plasma cells, lymphocytes or blasts expressing ASCT2)
f) concluding that the subject will achieve or is achieving a response with a modulator as described herein based on the correlation of the proportions determined in step a) with the reference value.

According to one embodiment of the present invention, the method as described herein enables to determine whether a subject suffering from a hematologic disorder will achieve a response with a modulator as described herein, *i.e.* enables to predict if a subject will achieve a response with a modulator as described herein before being treated with said modulator.

According to one embodiment of the present invention, the method as described herein enables to determine whether a subject suffering from a hematologic disorder is achieving a response with a modulator as described herein, *i*.*e*. enables to monitor whether a subject achieves a response when treated with a modulator as described herein.

It will be understood by the skilled artisan in the art that the monitoring of the response of the subject to a modulator as described herein enables to monitor the resistance of the hematologic disorder to a modulator as described herein.

In other words, the present invention relates to a method for monitoring the resistance of a hematologic disorder to a modulator as described herein in a subject, comprising a step of determining i) the proportion of plasma cells, lymphocytes or blasts expressing GLUT1 or ii) the proportion of plasma cells, lymphocytes or blasts expressing ASCT2 or iii) both proportions, among total plasma cells, lymphocytes or blasts in a sample obtained from the subject.

The method as described herein may comprise the steps of:
a) determining the proportion of plasma cells, lymphocytes or blasts expressing GLUT1 among total plasma cells, lymphocytes or blasts in a sample obtained from the subject,
b) comparing the proportion of plasma cells, lymphocytes or blasts expressing GLUT1 determined in step a) with a reference value, and
c) concluding that the hematological disorder is resistant to a modulator as described herein based on the correlation of the proportion of plasma cells, lymphocytes or blasts expressing GLUT1 determined in step a) with the reference value.

The method as described herein may comprise the steps of:
a) determining the proportion of plasma cells, lymphocytes or blasts expressing ASCT2 among total plasma cells, lymphocytes or blasts in a sample obtained from the subject,
b) comparing the proportion determined in step a) with a reference value, and
c) concluding that the hematological disorder is resistant to a modulator as described herein based on the correlation of the proportion determined in step a) with the reference value.

The method as described herein may comprise the steps of:
a) determining the proportion of plasma cells, lymphocytes or blasts expressing GLUT1, and the proportion of plasma cells, lymphocytes or blasts expressing ASCT2 among total plasma cells, lymphocytes or blasts in a sample obtained from the subject,
b) comparing the proportions of plasma cells, lymphocytes or blasts determined in step a) with two reference values for GLUT1 and ASCT2 (i.e. one reference value for the proportion of plasma cells, lymphocytes or blasts expressing GLUT1, and one reference value for the proportion of plasma cells, lymphocytes or blasts expressing ASCT2), and
c) concluding that the hematological disorder is resistant to a modulator as described herein based on the correlation of the proportions determined in step a) with the reference values.

The hematologic disorder may be as described herein.

The hematologic disorder may be selected from the group comprising or consisting of leukemia, lymphoma, MM, plasmacytoma, MGUS, SMM, myelodysplastic syndromes (MDS) and myeloproliferative neoplasms (MPNs).

The hematologic disorder as described herein may be selected from the group comprising or consisting of leukemia, lymphoma and MM.

The hematologic disorder as described herein may be selected from the group comprising or consisting of MM, plasmacytoma, MGUS, SMM, acute myeloid leukemia (AML), acute and chronic lymphocytic leukemia (ALL and CLL), myelodysplastic syndromes (MDS), myeloproliferative neoplasms (MPNs) and relapsed/refractory mantle cell lymphoma (MCL).

The hematologic disorder as described herein may be, in particular, MGUS, plasmacytoma, SMM or MM, and more particularly, MGUS, SMM or MM.

The present invention relates to a method for determining whether a subject suffering from plasmacytoma or MM will achieve or is achieving a response with a modulator as described herein, comprising a step of determining the proportion of plasma cells expressing GLUT1, or ii) the proportion of plasma cells expressing ASCT2 or iii) both proportions, among total plasma cells in a sample obtained from the subject.

The present invention relates to a method for determining whether a subject suffering from leukemia, such as AML, ALL or CLL, will achieve or is achieving a response with a modulator as described herein, comprising a step of determining i) the proportion of blast cells expressing GLUT1, or ii) the proportion of blast cells expressing ASCT2 or iii) both proportions, among total blast cells in a sample obtained from the subj ect.

The present invention relates to a method for determining whether a subject suffering from MDS will achieve or is achieving a response with a modulator as described herein, comprising a step of determining i) the proportion of blast cells expressing GLUT1, or ii) the proportion of blast cells expressing ASCT2 or iii) both proportions, among total blast cells in a sample obtained from the subject.

The present invention relates to a method for determining whether a subject suffering from MPNs will achieve or is achieving a response with a modulator as described herein, comprising a step of determining i) the proportion of blast cells expressing GLUT1, or ii) the proportion of blast cells expressing ASCT2 or iii) both proportions, among total blast cells in a sample obtained from the subject.

The present invention relates to a method for determining whether a subject suffering from lymphoma, such as MCL, will achieve or is achieving a response with a modulator as described herein, comprising a step of determining i) the proportion of lymphocytes expressing GLUT1, or ii) the proportion of lymphocytes expressing ASCT2 or iii) both proportions, among total lymphocytes in a sample obtained from the subject.

According to one embodiment of the invention, the method as described herein is for determining whether a subject suffering from a hematologic disorder will achieve a response or is achieving a response with an inhibitor of the anti-apoptotic BCL-2 family proteins as described herein.

According to one embodiment of the invention, the method as described herein is for determining whether a subject suffering from a hematologic disorder will achieve a response or is achieving a response with an agent modulating phosphorylation or stability of the BCL-2 family proteins as described herein.

The modulator, in particular, the inhibitor or the agent, as described herein may inhibit anti-apoptotic BLC-2 family proteins function. The modular, in particular, the inhibitor or the agent, as described herein may inhibit the anti-apoptotic function of the anti-apoptotic BLC-2 family proteins.

As used herein, the term "inhibit anti-apoptotic BLC-2 family proteins function" refers to a decreased function as compared to a reference function induced by anti-apoptotic BLC-2 family proteins activation, such as, for example, a function inferior or equal to 90%, 80%, 70%, 60%, 50%, 40%, 30%, 20%, 10%, or less of the reference function. The inhibition of anti-apoptotic BLC-2 family proteins function may be measured after contacting a cell with a compound tested for its impact on function, and the reference function may correspond to a function induced by anti-apoptotic BLC-2 family proteins activation measured in a cell not contacted with said compound.

The modulator, in particular, the inhibitor or the agent, as described herein may inhibit anti-apoptotic BLC-2 family proteins expression. Said inhibition may be done, for example, by reducing the transcription of the genes coding for the anti-apoptotic BLC-2 family proteins, or by degrading the anti-apoptotic BLC-2 family proteins.

As used herein, the term "inhibit anti-apoptotic BLC-2 family proteins expression" refers to a decreased expression level as compared to a reference expression level of anti-apoptotic BLC-2 family proteins, such as, for example, a level inferior or equal to 90%, 80%, 70%, 60%, 50%, 40%, 30%, 20%, 10%, or less of the reference expression level. The inhibition of anti-apoptotic BLC-2 family proteins expression may be measured after contacting a cell with a compound tested for its impact on expression, and the reference expression level may correspond to an expression level of anti-apoptotic BLC-2 family proteins measured in a cell not contacted with said compound.

As used herein, a subject may be considered as achieving a response with a modulator as described herein if the subject presents at least one of the following ameliorations when treated with a modulator as described herein: slowing down or stopping the progression, aggravation, or deterioration of one or more symptoms of the hematologic disorder; ameliorations of the symptoms of the hematologic disorder; reduction of the severity of the hematologic disorder; and reduction of the number of tumor cells.

The BCL-2 family refers to a family of proteins that regulate apoptosis. This family comprises pro-apoptotic and anti-apoptotic proteins. As used herein, "anti-apoptotic BCL-2 family proteins" refers to the anti-apoptotic proteins from the BCL-2 family, *i.e.* BCL-2, BCL-W, BCL-XL, MCL-1, A1, and BFL-1.

In humans, BCL-2 protein is encoded by the *BCL2* gene. An example of a human BCL-2 is represented by the Uniprot reference P10415.

As used herein, an inhibitor of anti-apoptotic BCL-2 family proteins refers to a substance inducing an inhibition or down-regulation of a biological activity associated with activation of anti-apoptotic BCL-2 family proteins, including any of the downstream biological effects otherwise resulting from the binding of the anti-apoptotic BCL-2 family proteins to their natural ligands.

The inhibitor of anti-apoptotic BCL-2 family proteins as described herein may be selected from the group comprising or consisting of an inhibitor of BCL-2, an inhibitor of BCL-W, an inhibitor of BCL-XL, an inhibitor of MCL-1, an inhibitor of A1, and an inhibitor of BFL-1, and a combination thereof.

It will be understood by the skilled artisan in the art that some inhibitors as described herein may be selective for one protein of the anti-apoptotic BCL-2 family, *i.e.* the inhibitor has an affinity for one protein of the anti-apoptotic BCL-2 family which is at least 10-fold, 25-fold, 50-fold, 75-fold, 80-fold, 90-fold, 95 fold, 100-fold, 125-fold, 150-fold, 200-fold, 250-fold, 300-fold, 350-fold, 400-fold, 450-fold, or 500-fold higher than the affinity for the other proteins of the anti-apoptotic BCL-2 family.

It will be understood by the skilled artisan in the art that some inhibitors may be selective for more than one protein of the anti-apoptotic BCL-2 family, such as two or three proteins of the anti-apoptotic BCL-2 family.

The inhibitor the anti-apoptotic BCL-2 family proteins as described herein may be selected from the group comprising or consisting of small organic molecules, polypeptides and proteins, and nucleic acids.

The inhibitor as described herein may be an inhibitor of BCL-2. In particular, the inhibitor may be selected from the group comprising or consisting of venetoclax (ABT-199), S55746 (BLC201), APG-2575 (lisaftoclax), DC-B01, oblimersen and DT2216.

The inhibitor as described herein may be an inhibitor of BCL-2 and BCL-XL. In particular, the inhibitor may be selected from the group comprising or consisting of AZD4320, AZD0466, APG-1252 (pelcitoclax), LP-118, BM-1197, S44563, 753b and WH244.

The inhibitor as described herein may be an inhibitor of BCL-2, BCL-XL and BCL-W. In particular, the inhibitor may be selected from the group comprising or consisting of ABT-737 and ABT-263 (navitoclax).

The inhibitor as described herein may be an inhibitor of BCL-2, BCL-XL, BCL-W and MCL-1. In particular, the inhibitor may be selected from the group comprising or consisting of obatoclax (GX15-070) and R-(-)-gossypol acetic acid (AT101).

The inhibitor as described herein may be an inhibitor of MCL-1. In particular, the inhibitor may be selected from the group comprising or consisting of A1210477 and S63845.

The inhibitor as described herein may be an inhibitor of BCL-2, in particular, venetoclax.

Formula of some compounds are presented in the table 3 below.

**Table 3**

| | |
|---|---|
| Venetoclax | 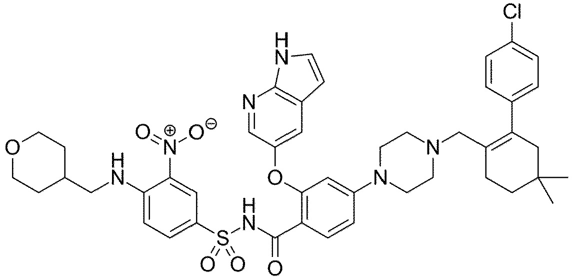 |
| Obatoclax | 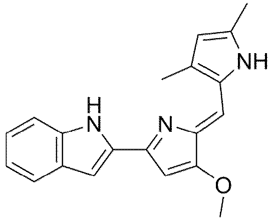 |
| Navitoclax | 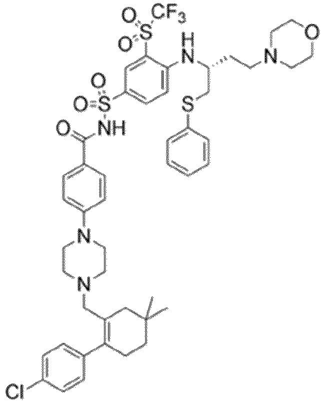 |
| Pelcitoclax | 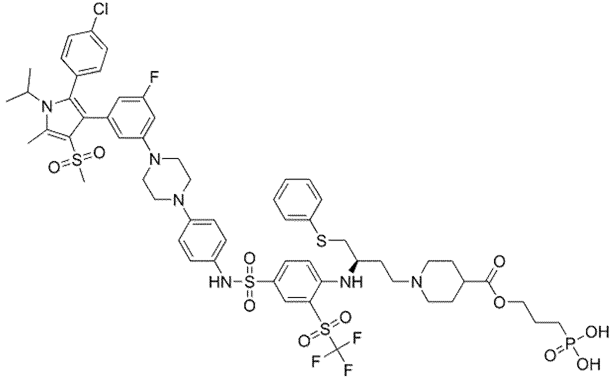 |
| AT-101 | 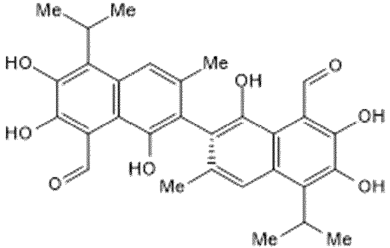 |
| ABT-737 | 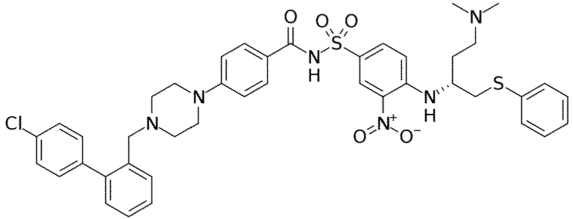 |
| S55746 | 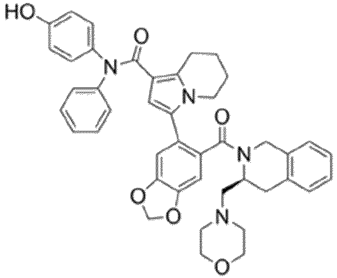 |
| APG-2575 | 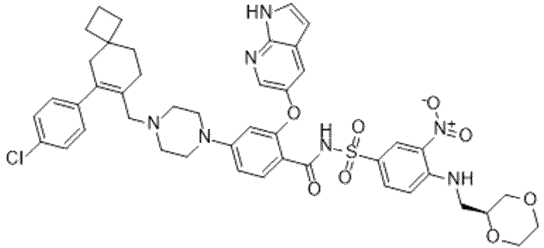 |
| AZD4320 | 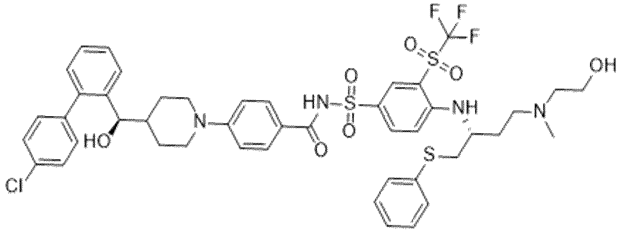 |
| BM-1197 | 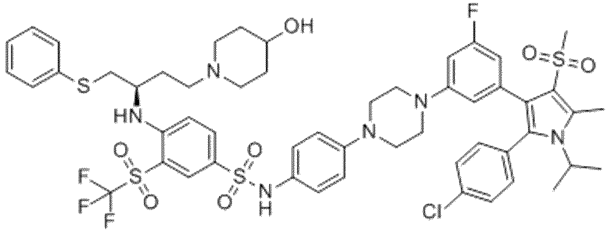 |
| S44563 | 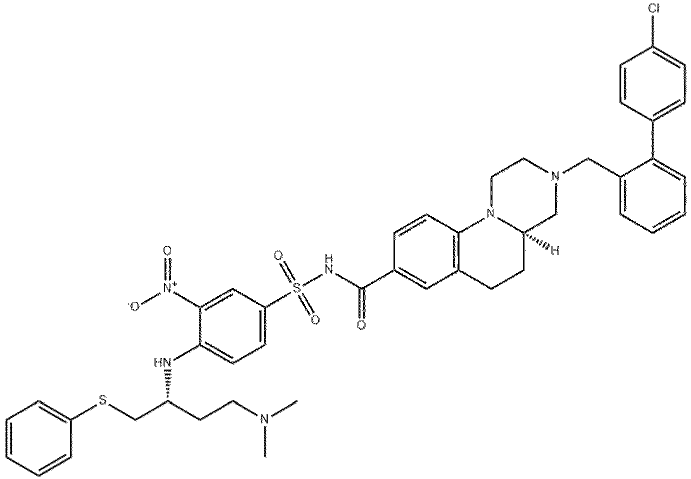 |
| A1210477 | 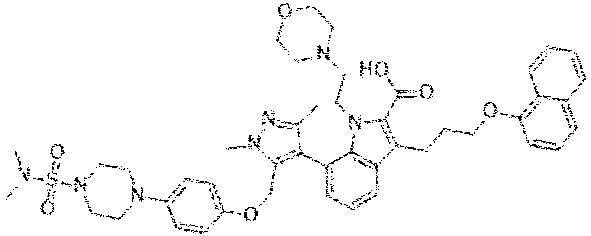 |
| S63845 | 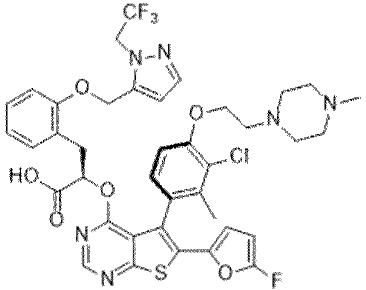 |
| DT2216 | 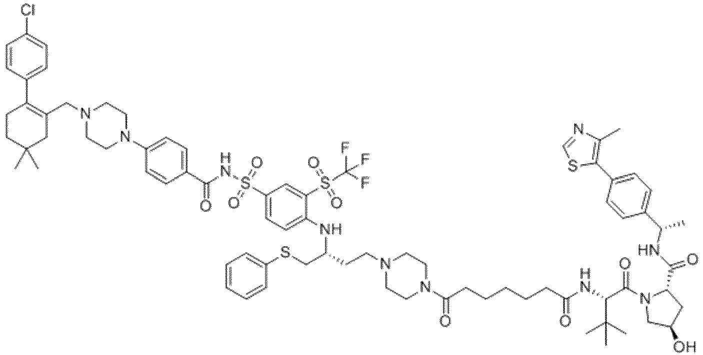 |
| 753b | 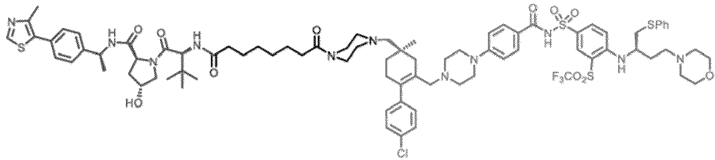 |

The sequence of oblimersen is: 5'-tct ccc age gtg cgc cat-3' (SEQ ID NO: 1).

Other examples of inhibitors of anti-apoptotic BCL-2 family proteins include, without limitation, alkaloids such as noscapine, papaverine, evodiamine, phaeanthine, isorhynchophylline, maritoclax; phenanthrenes including erianthridin, ephemeranthol A, flavonoids including luteolin, apigenin, quercetin, cardamonin, dihydromyricetin, gambogenic acid; phenolic including bergenin, curcumin; naphthoquinone including ø,ø-Dimethylacrylshikonin; terpenoid including ursolic acid, pseudolaric acid B and andrographolide.

Without being bound by any theory, it is hypothesized that phaeanthine, isorhynchophylline, ephemeranthol A, quercetin, cardamonin, curcumin, ursolic acid, pseudolaric acid B and andrographolide inhibit BCL-2, BCL-XI, and MCL-1 by inhibiting AKT function.

Without being bound by any theory, it is hypothesized that isorhynchophylline, cardamonin, and pseudolaric acid B inhibit BCL-2, BCL-XI, and MCL-1 by inhibiting STAT3 function.

Without being bound by any theory, it is hypothesized that noscapine, papaverine, evodiamine inhibit BCL-2, BCL-XI, and MCL-1 by inhibiting NF-xB function.

Without being bound by any theory, it is hypothesized that isorhynchophylline, erianthridin, and pseudolaric acid B inhibit BCL-2, BCL-XI, and MCL-1 by inhibiting ERK function.

Without being bound by any theory, it is hypothesized that gambogenic acid inhibits BCL-2, BCL-XI, and MCL-1 by promoting p53 and/or JNK function.

As used herein, an agent modulating phosphorylation of the BCL-2 family proteins refers to a substance modulating the level of phosphorylation/dephosphorylation of BCL-2 family proteins. Said agent may modulate the activity of kinases or phosphatases regulating the phosphorylation/dephosphorylation of BCL-2 family proteins, such as for example, protein phosphatase 2A (PP2A).

Means for assessing the level of phosphorylation/dephosphorylation are well known to the skilled artisan in the art and include, without limitation, Western blots.

Examples of such agents, include, without limitation, phosphatase-activating drugs, including FTY720.

As used herein, an agent modulating the stability of the BCL-2 family proteins refers to a substance modulating the stability of BCL-2 family proteins, such as, for example, modulating OTUD1 (ovarian tumor protease (OTU) domain-containing protein 1), which regulates the stability of BCL-2 family proteins, in particular BALF1.

According to one embodiment of the invention, the method as described herein comprises a step of comparing the proportion of plasma cells, lymphocytes or blasts expressing GLUT1, and/or the proportion of plasma cells, lymphocytes or blasts expressing ASCT2, with one or more reference value(s).

The reference value may be determined as described hereinabove.

The reference value may be determined in a reference population. The reference population of subjects may comprise at least 40 subjects, at least 100 subjects, at least 500 subjects, at least 1000 subjects, or more subjects.

The reference value may be the proportion of plasma cells, lymphocytes or blasts expressing GLUT1 or ASCT2 among total plasma cells, lymphocytes or blasts determined in a population of subjects suffering from a hematologic disorder. It may be concluded that the subject will achieve or is achieving a response with a modulator as described herein when:
- the proportion of plasma cells, lymphocytes or blasts expressing GLUT1 determined in step a) is higher than the reference value, when the method comprises the determination of only the proportion of plasma cells, lymphocytes or blasts expressing GLUT1, or
- the proportion of plasma cells, lymphocytes or blasts expressing ASCT2 determined in step a) is lower than the reference value, when the method comprises the determination of only the proportion of plasma cells, lymphocytes or blasts expressing ASCT2,
- the proportion of plasma cells, lymphocytes or blasts expressing GLUT1 determined in step a) is higher than the reference value, and the proportion of plasma cells, lymphocytes or blasts expressing ASCT2 determined in step a) is lower than the reference value, when the method comprises the determination of the proportion of plasma cells, lymphocytes or blasts expressing GLUT1 and the proportion of plasma cells, lymphocytes or blasts expressing ASCT2.

The reference value may be the proportion of plasma cells, lymphocytes or blasts expressing GLUT1 or ASCT2 among total plasma cells, lymphocytes or blasts determined in a population of subjects suffering from a hematologic disorder and achieving a response with a modulator as described herein. It may be concluded that the subject will achieve or is achieving a response with a modulator as described herein when:
- the proportion of plasma cells, lymphocytes or blasts expressing GLUT1 determined in step a) is equal or higher than the reference value, when the method comprises the determination of only the proportion of plasma cells, lymphocytes or blasts expressing GLUT1, or
- the proportion of plasma cells, lymphocytes or blasts expressing ASCT2 determined in step a) is equal or lower than the reference value, when the method comprises the determination of only the proportion of plasma cells, lymphocytes or blasts expressing ASCT2, or
- the proportion of plasma cells, lymphocytes or blasts expressing GLUT1 determined in step a) is equal or higher than the reference value, and the proportion of plasma cells, lymphocytes or blasts expressing ASCT2 determined in step a) is equal or lower than the reference value, when the method comprises the determination of the proportion of plasma cells, lymphocytes or blasts expressing GLUT1 and the proportion of plasma cells, lymphocytes or blasts expressing ASCT2.

The reference value may be the proportion of plasma cells, lymphocytes or blasts expressing GLUT1 or ASCT2 among total plasma cells, lymphocytes or blasts determined in a population of subjects suffering from a hematologic disorder and not achieving a response with a modulator as described herein. It may be concluded that the subject will achieve or is achieving a response with a modulator as described herein when:
- the proportion of plasma cells, lymphocytes or blasts expressing GLUT1 determined in step a) is higher than the reference value when the method comprises the determination of only the proportion of plasma cells, lymphocytes or blasts expressing GLUT1, or
- the proportion of plasma cells, lymphocytes or blasts expressing ASCT2 determined in step a) is lower than the reference value when the method comprises the determination of only the proportion of plasma cells, lymphocytes or blasts expressing ASCT2, or
- the proportion of plasma cells, lymphocytes or blasts expressing GLUT1 determined in step a) is higher than the reference value, and the proportion of plasma cells, lymphocytes or blasts expressing ASCT2 determined in step a) is lower than the reference value, when the method comprises the determination of the proportion of plasma cells, lymphocytes or blasts expressing GLUT1 and the proportion of plasma cells, lymphocytes or blasts expressing ASCT2.

The reference proportion of plasma cells, lymphocytes or blasts expressing GLUT1 among total plasma cells, lymphocytes or blasts may be a value comprised from about 10% to about 45%, preferably from about 30% to about 40%. In particular, the reference proportion of plasma cells, lymphocytes or blasts expressing GLUT1 may be one the following values: about 10%, 15%, 20%, 25%, 30%, 35%, 40% or about 45%, preferably about 35%.

The reference value may also be a personalized reference, determined earlier in the same subject. In particular, the reference value may be:
- the proportion of plasma cells, lymphocytes or blasts expressing GLUT1 among total plasma cells, lymphocytes or blasts determined earlier in the subject, when the method comprises the determination of only the proportion of plasma cells, lymphocytes or blasts expressing GLUT1, or
- the proportion of plasma cells, lymphocytes or blasts expressing ASCT2 among total plasma cells, lymphocytes or blasts determined earlier in the subject, when the method comprises the determination of only the proportion of plasma cells, lymphocytes or blasts expressing ASCT2, or
- the proportion of plasma cells, lymphocytes or blasts expressing GLUT1 among total plasma cells, lymphocytes or blasts and the proportion of plasma cells, lymphocytes or blasts expressing ASCT2 among total plasma cells, lymphocytes or blasts determined earlier in the subject, when the method comprises the determination of the proportion of plasma cells, lymphocytes or blasts expressing GLUT1 and the proportion of plasma cells, lymphocytes or blasts expressing ASCT2.

It may be concluded that the subject is achieving a response with a modulator as described herein when:
- the proportion of plasma cells, lymphocytes or blasts expressing GLUT1 determined in step a) is higher than the reference value, when the method comprises the determination of only the proportion of plasma cells, lymphocytes or blasts expressing GLUT1, or
- the proportion of plasma cells, lymphocytes or blasts expressing ASCT2 determined in step a) is lower than the reference value, when the method comprises the determination of only the proportion of plasma cells, lymphocytes or blasts expressing ASCT2, or
- the proportion of plasma cells, lymphocytes or blasts expressing GLUT1 determined in step a) is higher than the reference value and the proportion of plasma cells, lymphocytes or blasts expressing ASCT2 determined in step a) is lower than the reference value, when the method comprises the determination of the proportion of plasma cells, lymphocytes or blasts expressing GLUT1 and the proportion of plasma cells, lymphocytes or blasts expressing ASCT2.

When the method as described herein comprises the determination of only the proportion of plasma cells, lymphocytes or blasts expressing GLUT1, it may be concluded that the subject will achieve or is achieving a response with a modulator as described herein when the proportion of plasma cells, lymphocytes or blasts expressing GLUT1 determined in step a) is about 10% or more higher than the second one, such as about 20, 30, 40, 50, 60, 70, 80, 90% or more higher than the second one.

When the method as described herein comprises the determination of only the proportion of plasma cells, lymphocytes or blasts expressing ASCT2, it may be concluded that the subject will achieve or is achieving a response with a modulator as described herein when the proportion of plasma cells, lymphocytes or blasts expressing ASCT2 determined in step a) is about 10% or more lower than the second one, such as about 20, 30, 40, 50, 60, 70, 80, 90% or more lower than the second one.

When the method as described herein comprises the determination of the proportion of plasma cells, lymphocytes or blasts expressing GLUT1 and the proportion of plasma cells, lymphocytes or blasts expressing ASCT2, it may be concluded that the subject will achieve or is achieving a response with a modulator as described herein when:
- the proportion of plasma cells, lymphocytes or blasts expressing GLUT1 determined in step a) is about 10% or more higher than the second one, such as about 20, 30, 40, 50, 60, 70, 80, 90% or more higher than the second one, and
- the proportion of plasma cells, lymphocytes or blasts expressing ASCT2 determined in step a) is about 10% or more lower than the second one, such as about 20, 30, 40, 50, 60, 70, 80, 90% or more lower than the second one.

The proportion of plasma cells, lymphocytes or blasts expressing GLUT1 or ASCT2 may be determined by measuring the expression levels of GLUT1 and/or ASCT2.

As used herein, the term "expression" may refer alternatively to the transcription *(i.e.* expression of the RNA) or to the translation *(i.e.* expression of the protein) of GLUT1 and/or ASCT2, or to the presence of GLUT 1 and/or ASCT2 at the surface of the cell.

Methods for determining the expression level are well-known from the skilled artisan, and include, without limitation, determining the transcriptome (in particular related to transcription of GLUT1 or ASCT2) or the proteome (in particular related to translation of GLUT1 or ASCT2) of a cell.

The expression of GLUT1 and/or ASCT2 may be assessed at the RNA level.

Methods for assessing the transcription level of a transporter are well known in the prior art. Examples of such methods include, but are not limited to, RT-PCR, RT-qPCR, Northern Blot, hybridization techniques such as, for example, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like.

The expression of GLUT1 and/or ASCT2 may be assessed at the protein level.

*In vitro* methods for determining a protein level in a sample are well-known in the art. Examples of such methods include, but are not limited to, immunohistochemistry, Multiplex methods (Luminex), Western blot, enzyme-linked immunosorbent assay (ELISA), sandwich ELISA, fluorescent-linked immunosorbent assay (FLISA), enzyme immunoassay (EIA), radioimmunoassay (RIA), flow cytometry (FACS) and the like.

*In vivo* methods for determining a protein level are well-known in the art. Examples of such methods include, but are not limited to, computed tomography (CT scan), endoscopic ultrasound (EUS), magnetic resonance imaging (MRI), positron-emission tomography (PET), single photon emission tomography (SPECT), magnetic resonance cholangiopancreatography, fluorimetry, fluorescence, and near-infrared (NIR) fluorescent imaging.

The determination of the expression levels of GLUT1 and/or ASCT2 may correspond to the detection and/or quantification of said transporter on the cell surface.

Methods for analyzing the presence of a protein on the cell surface are well-known to the skilled artisan and include, without limitation, FACS analysis, immunohistochemistry, western blot associated with cell fractionation, enzyme-linked immunosorbent assay (ELISA), sandwich ELISA, fluorescent-linked immunosorbent assay (FLISA), enzyme immunoassay (EIA), radioimmunoassay (RIA), image analysis, for example high content analysis, computed tomography (CT scan), endoscopic ultrasound (EUS), magnetic resonance imaging (MRI), positron-emission tomography (PET), single photon emission tomography (SPECT), magnetic resonance cholangiopancreatography, fluorimetry, fluorescence, and near-infrared (NIR) fluorescent imaging and the like.

In some embodiments, it is considered that a cell does not express GLUT1 or ASCT2 if the expression levels of GLUT 1 or ASCT2 are lower than two-fold, three-fold, four-fold, or five-fold the background noise.

In some embodiments, it is considered that a cell expresses GLUT1 or ASCT2 if the expression levels of GLUT1 or ASCT2 are higher than two-fold, three-fold, four-fold, five-fold or ten-fold the background noise.

In some embodiments, it is considered that a cell does not express GLUT1 or ASCT2 if the expression levels of GLUT1 or ASCT2 are lower than five-fold the background noise and that a cell expresses GLUT1 or ASCT2 if the expression levels of GLUT1 or ASCT2 are higher than ten-fold the background noise.

The determination of the expression levels of GLUT1 and/or ASCT2 may correspond to the detection and/or quantification of the binding of a ligand to GLUT1 or ASCT2.

As used herein, the term "ligand" refers to any substance that forms a complex with GLUT1 or ASCT2. Typical ligands include, but are not limited to, polypeptides and proteins.

Said ligand may be a receptor-binding domain ligand and the method as described herein comprises detecting and/or quantifying a complex formed between said receptor-binding domain ligand and GLUT1 or ASCT2.

Said ligand may be an antibody specific of GLUT1 or ASCT2, and the method as described herein comprises detecting and/or quantifying a complex formed between said antibody and GLUT1 or ASCT2.

The expression "detecting and/or quantifying binding of a ligand, such as, for example, a receptor-binding domain ligand, to GLUT1 or ASCT2" means that when GLUT1 or ASCT2 is present, a complex is formed between the receptor and the ligand.

The complex can be detected if the ligand has been, for example, but not limited to, covalently coupled with a detectable molecule such as an antibody constant fragment (Fc) or a fluorescent compound (e.g. Fluorescent proteins Cyanine dye, Alexa dye, Quantum dye, etc). The complex can also be detected if the ligand has been tagged with different means well known to the person skilled in the art. For example, but without limitation, a tag used in the invention can be a tag selected from the group comprising or consisting of Hemagglutinin Tag, Poly Arginine Tag, Poly Histidine Tag, Myc Tag, Strep Tag, S-Tag, HAT Tag, 3x Flag Tag, Calmodulin-binding peptide Tag, SBP Tag, Chitin binding domain Tag, GST Tag, Maltose-Binding protein Tag, Fluorescent Protein Tag, T7 Tag, V5 Tag and Xpress Tag. The complex can also be detected by the use of an antibody directed against the ligand. Said antibody directed against the ligand may be coupled or fused to a detectable label, tag or contrast agent. The use of the ligand therefore allows on the one hand the identification and detection of the cell surface transporter depending on the ligand used, and on the other hand the quantification of the complex formed.

Detection and/or quantification of binding may be conducted by flow cytometry, immunofluorescence or image analysis, for example, high content analysis.

The complex can be detected if the ligand has been for example, but not limited to, covalently coupled with at least one contrast agent. Detecting and/or quantification of binding may be conducted by medical imaging technics.

As used herein, the term "contrast agent" refers to agents used to improve the visibility of internal bodily structures in medical imaging technics, including, but not limited to, magnetic resonance imaging (MRI), X-ray-based imaging techniques such as computed tomography (CT), radiography, endoscopic ultrasound (EUS), positron-emission tomography (PET), single photon emission tomography (SPECT), magnetic resonance cholangiopancreatography, fluoroscopy, fluorimetry, fluorescence, and near-infrared (NIR) fluorescent imaging.

The ligand may be coupled with at one least contrast agent, wherein said contrast agent may be a radiolabeled agent or a fluorescent agent.

The radiolabeled agent as described herein may be selected from the group comprising or consisting of a non-metallic radioisotope, non-metallic or metallic dye, paramagnetic metal, or radioactive metal.

Examples of non-metallic radioisotopes comprise but are not limited to: I-125, I-123, I-131, C-11, F-18, Br-75, Br-76, Br-77, Br-80, and At-211. The non-metallic radioisotopes may be conjugated covalently to either terminus of the ligand, functional groups of amino acid side chains, be part of a linear stabilized peptide as an additional substituent, e.g. in an amino acid phenylalanine or tyrosine carrying fluorine, bromine or iodine, or as an additional substituent carboxy or methyl, or as a replacement of any regular carbon atom in the ligand by 1-125. In particular, the ligand may be coupled with 1-125. These radioisotopes are useful in ligands as positron emission tomography (PET) probes or as single-photon emission computed tomography (SPECT) probes.

Examples of non-metallic or metallic dyes comprise, but are not limited to, organic molecules, e.g., commercial Alexa fluor dyes, fluorescein, rhodamine, or Cy5.5, complexes of transition metals, e.g. chelates of Eu³⁺, Tb³⁺, or nanoparticles (quantum dots) which adsorb and/or emit light in the visible range or in the near infrared. Organic dyes and chelating systems will be coupled to the ligands as described above for chelators. Conjugation of the ligands with quantum dots is done by procedures known to those skilled in the art. These ligands carrying dyes are useful as optical imaging probes.

Examples of paramagnetic metals comprise, but are not limited to, Gd, Fe, Mn. The metals are attached to the ligands. These ligands are useful as magnetic resonance imaging (MRI) probes.

Examples of radioactive metals comprise but are not limited to: Tc-99m, Ga-67, Ga-68, Lu-177, Cu-64, and Zr-89, Re-186/188, Bi-213, Y- 90, Cu-67, Lu-177, Tb-161, Tc-99m, and In-111. The radioactive metals (and the paramagnetic metals mentioned above) are attached to the ligands of the invention through chelators as listed above, directly connected to the ligands or through a spacer.

Examples of fluorescent agents include, but are not limited to, GFP, mPlum^{g}, mCherry^{g}, tdTomato^{g}, mStrawberry^{g}, J-Red, DS-Red monomer^{h}, mOrange^{g}, mKO, mCitrineⁱ, Venus, YPet^{g}, EYFP, Emerald^{g}, EGFP, CyPet, mCFP^{m}, Cerulean^{g}, T-Sapphire^{g}, indocyanine green, ZW800-1, Cy5.5 and IRDye800CW.

In particular, the contrast agent may be I-125.

The ligand may be a receptor-binding domain (RBD) ligand, wherein said RBD ligand comprises a part or the totality of a RBD derived from the soluble part of a glycoprotein of an enveloped virus that interacts with GLUT1 or ASCT2. The ligand may be soluble, *i.e.* it does not comprise a transmembrane domain, and is therefore not anchored to a membrane.

The expression "derived from the soluble part of the glycoprotein of an enveloped virus" means that the ligand is a fragment or a part of a glycoprotein contained in the envelope of a virus and can be obtained, for example, by cloning.

The term "glycoprotein" is to be understood as meaning an envelope glycoprotein, a coat glycoprotein or a fusion glycoprotein, wherein the term "glycoprotein" refers to a protein containing oligosaccharide chains covalently attached to polypeptide side-chains.

The expression "that interacts with GLUT1 or ASCT2" means that the glycoprotein is liable to recognize GLUT1 or ASCT2 present on the surface of the cell. Thus, a ligand that interacts with GLUT1 or ASCT2, will form a complex with said cell surface transporter, which complex may be detected by a method as here above described.

RBDs may be found, in particular, in glycoproteins of the envelope of viruses.

The RBD ligand may contain the total RBD or a fragment or part of the RBD.

The RBD ligand may be glycosylated or not glycosylated.

The ligand may comprise the SU domain of the glycoprotein envelope of a virus or a fragment of the SU domain, such as, for example, the RBD.

The ligand may not comprise the TM domain of the glycoprotein envelope of a virus. Therefore, the ligand may be a soluble peptide, such as, for example, a soluble RBD. As used herein, the term "soluble peptide" refers to a peptide which is not anchored within a membrane, such as, for example, by a transmembrane or a GPI anchor domain.

Said virus may be selected from the group comprising retroviruses, such as, for example, (i) gammaretroviruses such as for example, murine (MLV), feline (FeLV, RD114), avian (spleen necrosis virus (SNV), or simian baboon endogenous virus (BaEV), simian sarcoma virus (SSV), SSV-associated virus (SSaV) or betaretroviruses known to have (glyco)protein envelopes related to that of gammaretroviruses such as for example simian retrovirus (SRV) and Mason-Pfizer monkey virus (MPMV); and (ii) deltaretroviruses such as, for example, primate T cell leukaemia virus (PTLV) (such as, for example, human T cell leukaemia virus (HTLV) and simian T cell leukaemia virus (STLV)) and bovine leukemia virus (BLV).

The gamma and deltaretroviruses encode an Env glycoprotein present in mature retrovirus virions. The Env protein is synthesized in the form of a propeptide, which is dived in Golgi apparatus by furine peptidase, resulting in two polypeptides: the transmembrane (TM) and the cell surface (SU) components. The SU domain contains two major subdomains: a domain of interaction with the TM domain and the RBD, the further being liable to interact with host cell membrane receptors.

The ligand may be isolated from the glycoprotein of human endogenous retrovirus W, and is herein referred as HERV-W.RBD. The ligand may comprise a part or the totality of HERV-W.RBD and binds to ASCT1 and/or ASCT2.

Said HERV-W.RBD may comprises or consist of the amino acid sequence SEQ ID NO: 2, variants or fragments thereof. Said fragment may comprise or consist of amino acids 21 to 189 of SEQ ID NO: 2.

Said fragment may comprise or consist of amino acids 1 to 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 or 188 of SEQ ID NO: 2. Said fragment may comprise or consist of amino acids 21 to 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 or 188 of SEQ ID NO: 2.

Said HERV-W.RBD may comprise or consist of the amino acid sequence SEQ ID NO: 3, variants or fragments thereof. Said fragment comprises or consists of amino acids 21 to 189 of SEQ ID NO: 3.

Said fragment may comprise or consist of amino acids 1 to 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 or 188 of SEQ ID NO: 3. Said fragment may comprise or consist of amino acids 21 to 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 or 188 of SEQ ID NO: 3.

Said fragment may comprise or consist of SEQ ID NO: 4. Said fragment may comprise or consist of amino acids 21 to 121 of SEQ ID NO: 4.

Said HERV-W.RBD may comprise or consist of the amino acid sequence SEQ ID NO: 5, variants or fragments thereof. Said fragment may comprise or consist of amino acids 22 to 181 of SEQ ID NO: 5.

Said fragment may comprise or consist of amino acids 1 to 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, or 180 of SEQ ID NO: 5. Said fragment may comprise or consist of amino acids 22 to 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, or 180 of SEQ ID NO: 5.

The ligand may be isolated from the glycoprotein of feline endogenous virus, and is herein referred as RD114.RBD. The ligand may comprise a part or the totality of RD114.RBD and binds to ASCT1 and/or ASCT2.

Said RD114.RBD may comprise or consist of the amino acid sequence SEQ ID NO: 6 or fragments thereof. Said fragment may comprise or consist of amino acids 19 to 239 of SEQ ID NO: 6.

Said fragment may comprise or consist of amino acids 1 to 223, 224, 225, 226, 227, 228, 229, 230, 231, 232, 233, 234, 235, 236, 237 or 238 of SEQ ID NO: 6. Said fragment may comprise or consist of amino acids 19 to 223, 224, 225, 226, 227, 228, 229, 230, 231, 232, 233, 234, 235, 236, 237 or 238 of SEQ ID NO: 6.

Said fragment comprises or consists of SEQ ID NO: 7. Said fragment may comprise or consist of amino acids 19 to 222 of SEQ ID NO: 7.

Said fragment may comprise or consist of SEQ ID NO: 8. Said fragment may comprise or consist of amino acids 19 to 223 of SEQ ID NO: 8.

Said RD114.RBD may comprise or consist of the amino acid sequence SEQ ID NO: 9 or fragments thereof. Said fragment may comprise or consist of amino acids 22 to 331 of SEQ ID NO: 9.

Said fragment may comprise or consist of amino acids 1 to 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330 of SEQ ID NO: 9. Said fragment may comprise or consist of amino acids 22 to 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330 of SEQ ID NO: 9.

Said fragment may comprise or consist of SEQ ID NO: 9, or a fragment thereof. Said fragment may comprise or consist of amino acids 19 to 222 of SEQ ID NO: 9.

Said RD114.RBD may comprise or consist of the amino acid sequence SEQ ID NO: 10 or fragments thereof. Said fragment may comprise or consist of amino acids 1 to 222 of SEQ ID NO: 10.

Said fragment may comprise or consist of amino acids 1 to 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221 of SEQ ID NO: 10. Said fragment may comprise or consist of amino acids 19 to 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221 of SEQ ID NO: 10.

The ligand may be isolated from the glycoprotein of baboon endogenous virus, and is herein referred as BaEV.RBD. The ligand may comprise a part or the totality of BaEV.RBD and binds to ASCT1 and/or ASCT2.

Said BaEV.RBD may comprise or consist of the amino acid sequence SEQ ID NO: 11 or fragments thereof. Said fragment may comprise or consist of amino acids 19 to 563 of SEQ ID NO: 11.

Said fragment may comprise or consist of amino acids 1 to 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562 of SEQ ID NO: 11. Said fragment may comprise or consist of amino acids 19 to 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562 of SEQ ID NO: 11.

The ligand may be isolated from the glycoprotein of spleen necrosis virus, and is herein referred as SNV.RBD. The ligand may comprise a part or the totality of SNV.RBD and binds to ASCT1 and/or ASCT2.

Said SNV.RBD may comprise or consist of the amino acid sequence SEQ ID NO: 12 or fragments thereof.

Said fragment may comprise or consist of amino acids 1 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257 of SEQ ID NO: 12.

Said SNV.RBD may comprise or consist of the amino acid sequence SEQ ID NO: 13 or fragments thereof. Said fragment may comprise or consist of amino acids 37 to 567 of SEQ ID NO: 13.

Said fragment may comprise or consist of amino acids 1 to 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566 of SEQ ID NO: 13. Said fragment may comprise or consist of amino acids 37 to 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566 of SEQ ID NO: 13.

The ligand may be isolated from the glycoprotein of simian retrovirus, and is herein referred as SRV.RBD. The receptor-binding domain ligand may comprise a part or the totality of SRV.RBD and binds to ASCT1 and/or ASCT2.

Said SRV.RBD may comprise or consist of the amino acid sequence SEQ ID NO: 14 or fragments thereof. Said fragment may comprise or consist of amino acids 23 to 251 of SEQ ID NO: 14.

Said fragment may comprise or consist of amino acids 1 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250 of SEQ ID NO: 14. Said fragment may comprise or consist of amino acids 23 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250 of SEQ ID NO: 14.

The ligand may be isolated from the glycoprotein of Mason-Pfizer monkey virus, and is herein referred as MPMV.RBD. The ligand may comprise a part or the totality of MPMV.RBD and binds to ASCT1 and/or ASCT2.

Said MPMV.RBD may comprise or consist of the amino acid sequence SEQ ID NO: 15 or fragments thereof. Said fragment may comprise or consist of amino acids 23 to 250 of SEQ ID NO: 15.

Said fragment may comprise or consist of amino acids 1 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 of SEQ ID NO: 15. Said fragment may comprise or consist of amino acids 23 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 of SEQ ID NO: 15.

The ligand may be isolated or derived from the soluble part of an envelope glycoprotein of a human T-cell leukemia virus and binds to GLUT 1.

The ligand may comprise or consist of the total surface envelope protein (SU) of a human T-cell leukemia virus. The ligand may be a fragment of the surface envelope protein (SU) of a human T-cell leukemia virus.

The ligand may be isolated or derived from the soluble part of an envelope glycoprotein of human T-cell leukemia virus type 1 (HTLV-1) and binds to GLUT1.

The ligand may comprise or consist of the total surface envelope protein (SU) of human T-cell leukemia virus type 1 (HTLV-1), and preferably may have a sequence comprising or consisting of the amino acid sequence SEQ ID NO: 16, SEQ ID NO: 17 or a fragment or variant thereof.

The ligand may be a fragment of the surface envelope protein (SU) of human T-cell leukemia virus type 1 (HTLV-1), and preferably may have a sequence comprising or consisting of the amino acid sequence SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, or a fragment or variant thereof.

The ligand may be isolated or derived from the soluble part of an envelope glycoprotein of human T-cell leukemia virus type 2 (HTLV-2) and binds to GLUT1.

The ligand may comprise or consist of the total surface envelope protein (SU) of human T-cell leukemia virus type 2 (HTLV-2), and preferably may have a sequence comprising or consisting of the amino acid sequence SEQ ID NO: 27, SEQ ID NO: 28, or a fragment or variant thereof.

The ligand may be a fragment of the surface envelope protein (SU) of human T-cell leukemia virus type 2 (HTLV-2), and preferably may have a sequence comprising or consisting of the amino acid sequence SEQ ID NO: 29, SEQ ID NO: 30, SEQ ID NO: 31, SEQ ID NO: 32 or SEQ ID NO: 33, or a fragment or variant thereof.

The ligand may be isolated or derived from the soluble part of an envelope glycoprotein of human T-cell leukemia virus type 3 (HTLV-3) and binds to GLUT1.

The ligand may comprise or consist of the total surface envelope protein (SU) of human T-cell leukemia virus type 3 (HTLV-3), and preferably may have a sequence comprising or consisting of the amino acid sequence SEQ ID NO: 34, SEQ ID NO: 35, or a fragment or variant thereof.

The ligand may be a fragment of the surface envelope protein (SU) of human T-cell leukemia virus type 3 (HTLV-3), and preferably may have a sequence comprising or consisting of the amino acid sequence SEQ ID NO: 36, SEQ ID NO: 37, or a fragment or variant thereof.

The ligand may be isolated or derived from the soluble part of an envelope glycoprotein of human T-cell leukemia virus type 4 (HTLV-4) and binds to GLUT1.

The ligand may comprise or consist of the total surface envelope protein (SU) of human T-cell leukemia virus type 4 (HTLV-4), and preferably may have a sequence comprising or consisting of the amino acid sequence SEQ ID NO: 38, SEQ ID NO: 39 or a fragment or variant thereof.

The ligand may be a fragment of the surface envelope protein (SU) of human T-cell leukemia virus type 4 (HTLV-4), and preferably may have a sequence comprising or consisting of the amino acid sequence SEQ ID NO: 40 or SEQ ID NO: 41, SEQ ID NO: 42, SEQ ID NO: 43, or a fragment or variant thereof.

The ligand may be isolated or derived from the soluble part of an envelope glycoprotein of simian T-cell leukemia virus type 1 (STLV-1) and binds GLUT1.

The ligand may comprise or consist of the total surface envelope protein (SU) of simian T-cell leukemia virus type 1 (STLV-1), and preferably may have a sequence comprising or consisting of the amino acid sequence SEQ ID NO: 44, SEQ ID NO: 45 or a fragment or variant thereof.

The ligand may be a fragment of the surface envelope protein (SU) of simian T-cell leukemia virus type 1 (STLV-1), and preferably may have a sequence comprising or consisting of the amino acid sequence SEQ ID NO: 46, SEQ ID NO: 47, or a fragment or variant thereof.

The ligand may be isolated or derived from the soluble part of an envelope glycoprotein of simian T-cell leukemia virus type 2 (STLV-2) and binds GLTU1.

The ligand may comprise or consist of the total surface envelope protein (SU) of simian T-cell leukemia virus type 2 (STLV-2), and preferably may have a sequence comprising or consisting of the amino acid sequence SEQ ID NO: 48, SEQ ID NO: 49, or a fragment or variant thereof.

The ligand may be a fragment of the surface envelope protein (SU) of simian T-cell leukemia virus type 2 (STLV-2), and preferably may have a sequence comprising or consisting of the amino acid sequence SEQ ID NO: 50, SEQ ID NO: 51, or a fragment or variant thereof.

The ligand may be isolated or derived from the soluble part of an envelope glycoprotein of simian T-cell leukemia virus type 3 (STLV-3) and binds GLUT1.

The ligand may comprise or consist of the total surface envelope protein (SU) of simian T-cell leukemia virus type 3 (STLV-3), and preferably may have a sequence comprising or consisting of the amino acid sequence SEQ ID NO: 52, SEQ ID NO: 53, or a fragment or variant thereof.

The ligand may be a fragment of the surface envelope protein (SU) of simian T-cell leukemia virus type 3 (STLV-3), and preferably may have a sequence comprising or consisting of the amino acid sequence SEQ ID NO: 54, SEQ ID NO: 55, or a fragment or variant thereof.

The ligand may be isolated or derived from the soluble part of an envelope glycoprotein of simian T-cell leukemia virus type 5 (STLV-5).

The ligand may comprise or consist of the total surface envelope protein (SU) of simian T-cell leukemia virus type 5 (STLV-5), and preferably may have a sequence comprising or consisting of the amino acid sequence SEQ ID NO: 56, SEQ ID NO: 57, or a fragment or variant thereof.

Preferably, the ligand may be a fragment of the surface envelope protein (SU) of simian T-cell leukemia virus type 3 (STLV-3) and binds to GLUT1.

Preferably, the ligand may be isolated or derived from the soluble part of an envelope glycoprotein of T-cell leukemia virus type 2 (HTLV-2) and binds to GLUT1. Preferably, the ligand may have a sequence comprising or consisting of the amino acid sequence SEQ ID NO: 58, herein referred to as HTLV2.RBD or H2.RBD.

The ligands as described herein may comprise naturally standard amino acids or non-standard amino acids. Polypeptide mimetics include polypeptides having the following modifications: i) polypeptides wherein one or more of the peptidyl -C(O)NR-linkages (bonds) have been replaced by a non-peptidyl linkage such as a -CH₂-carbamate linkage (-CH₂OC(O)NR-), a phosphonate linkage, a -CH₂-sulfonamide (-CH₂-S(O)₂NR-) linkage, a urea (-NHC(O)NH-) linkage, a -CH₂-secondary amine linkage, or with an alkylated peptidyl linkage (-C(O)NR-) wherein R is C₁-C₄ alkyl; ii) polypeptides wherein the N-terminus is derivatized to a -NRR¹ group, to a -NRC(O)R group, to a -NRC(O)OR group, to a -NRS(O)₂R group, to a -NHC(O)NHR group where Rand R¹ are hydrogen or C₁-C₄ alkyl with the proviso that R and R¹ are not both hydrogen; iii) polypeptides wherein the C terminus is derivatized to -C(O)R² where R² is selected from the group consisting of C₁-C₄ alkoxy, and -NR³R⁴ where R³ and R⁴ are independently selected from the group consisting of hydrogen and C₁-C₄ alkyl.

When it is desired to alter the amino acid sequence of a polypeptide to create an equivalent, or even an improved, variant or portion of a ligand, one skilled in the art will typically change one or more of the codons of the encoding DNA sequence. For example, certain amino acids may be substituted by other amino acids in a protein structure without appreciable loss of its ability to bind cell surface receptor, preferably GLUT1 or ASCT2. Since it is the binding capacity and nature of a protein that defines that protein's biological functional activity, certain amino acid sequence substitutions can be made in a protein sequence, and, of course, its underlying DNA coding sequence, and nevertheless obtain a protein with similar properties. It is thus contemplated that various changes may be made in the peptide sequences, or corresponding DNA sequences that encode said peptides without appreciable loss of their biological utility or activity. In many instances, a polypeptide variant will contain one or more conservative substitutions.

A variant may also, or alternatively, contain nonconservative changes. In a preferred embodiment, variant polypeptides differ from a native sequence by substitution, deletion or addition of 1, 2, 3, 4, 5, 6, 7, 8, 9 or 10 amino acids. Variants may also (or alternatively) be modified by, for example, the deletion or addition of amino acids that have minimal influence on the immunogenicity, secondary structure and hydropathic nature of the polypeptide.

The ligands as described herein may be modified by means well-known in the art, for instance by the addition of one or more functional group such as a phosphate, acetate, lipid or carbohydrate group, and/or by the addition of one or more protecting group.

For example, the ligands can be modified by the addition of one or more functional groups such as phosphate, acetate, or various lipids and carbohydrates. The RBD ligands can also exist as polypeptide derivatives. The term "polypeptide derivative" refers to compound having an amino group (--NH--), and more particularly, a peptide bond. Polypeptides may be regarded as substituted amides. Like the amide group, the peptide bond shows a high degree of resonance stabilization. The C--N single bond in the peptide linkage has typically about 40 percent double-bond character and the C=O double bond about 40 percent single-bond character. "Protecting groups" are those groups that prevent undesirable reactions (such as proteolysis) involving unprotected functional groups. Specific examples of amino protecting groups include formyl; trifluoroacetyl; benzyloxycarbonyl; substituted benzyloxycarbonyl such as (ortho- or para-) chlorobenzyloxycarbonyl and (ortho- or para-) bromobenzyloxycarbonyl; and aliphatic oxycarbonyl such as t-butoxycarbonyl and t-amiloxycarbonyl. The carboxyl groups of amino acids can be protected through conversion into ester groups. The ester groups include benzyl esters, substituted benzyl esters such as methoxybenzyl ester; alkyl esters such as cyclohexyl ester, cycloheptyl ester or t-butyl ester. The guanidino moiety may be protected by nitro; or arylsulfonyl such as tosyl, methoxybenzensulfonyl or mesitylenesulfonyl, even though it does not need a protecting group. The protecting groups of imidazole include tosyl, benzyl and dinitrophenyl. The indole group of tryptophan may be protected by formyl or may not be protected.

The modification of the ligands aims in particular to improve their life time in vivo. One type of modification is the addition to the N or C termini of the RBD ligands of polyethylene glycol (PEG). PEG is known by the person skilled in the art to have many properties that make it an ideal carrier for polypeptides such as high water solubility, high mobility in solution and low immunogenicity. This modification also protects the polypeptides from exopeptidases and therefore increases their overall stability in vivo.

The other modifications used to prevent degradation of the polypeptides by endopeptidases or exopeptidases include N-terminal modifications such as acetylation or glycosylation, C-terminal modifications such as amidation and use of unnatural amino acids (β-amino and α-trifluoromethyl amino acids) at particular sites within the polypeptides.

The ligands as described herein may be fused to a detection tag or label. Examples of detection tag and labels are provided hereinabove.

The ligand as described herein may be fused to an Fc fragment from rabbit, mouse, human, monkey or other mammals or to albumin.

Examples of Fc fragments include, but are not limited to, rabbit Fc fragment (such as e.g. amino acid sequence SEQ ID NO: 59), mouse Fc fragment (such as e.g. amino acid sequence SEQ ID NO: 60), human Fc fragment or monkey Fc fragment.

The ligand may be HERV-W.RBD fused to a mouse Fc fragment, and preferably may have a sequence with SEQ ID NO: 61. The ligand may be RD114.RBD fused to a mouse Fc fragment, and preferably may have a sequence with SEQ ID NO: 62; or SEQ ID NO: 63.

The ligand may be HTLV2.RBD fused to a rabbit Fc fragment, for instance via a Gly/Ser linker, and preferably may have a sequence with SEQ ID NO: 64 or SEQ ID NO: 65. The ligand may be HTLV2.RBD fused to a mouse Fc fragment, for instance via a Gly/Ser linker, and preferably may have a sequence with SEQ ID NO: 66 or SEQ ID NO: 67.

The ligand as described herein may be fused to a label, and in particular, a fluorescent agent.

The ligand may be HTLV2.RBD fused to a GFP, for instance via a Gly/Ser linker, and preferably may have a sequence with SEQ ID NO: 68 or SEQ ID NO: 69.

The present invention also relates to a method for treating a subject suffering from a hematologic disorder, comprising the steps of determining whether the subject will achieve a response with a modular as described herein with a method as described hereinabove, and treating said subject.

The method as described may be used to treat a subject with an adapted treatment by determining, before treatment, the responsiveness of the subject suffering from a hematologic disorder to a modulator as described herein.

The method as described herein may comprise the steps of:
a) determining i) the proportion of plasma cells, lymphocytes or blasts expressing GLUT1 or ii) the proportion of plasma cells, lymphocytes or blasts expressing ASCT2, among total plasma cells, lymphocytes or blasts in a sample obtained from the subject,
b) comparing the proportion determined in step a) with a reference value,
c) predicting whether the subject will achieve a response with a modulator as described herein based on the correlation of the proportion determined in step a) with the reference value, and
d) treating the subject with a modulator as described herein if said subject has been predicted to achieve a response with said modulator.

The method as described herein may comprise the steps of:
a) determining the proportion of plasma cells, lymphocytes or blasts expressing GLUT1, and the proportion of plasma cells, lymphocytes or blasts expressing ASCT2, among total plasma cells, lymphocytes or blasts in a sample obtained from the subject,
b) comparing the proportions determined in step a) with two reference values for GLUT1 and ASCT2 *(i.e.* one reference value for the proportion of plasma cells, lymphocytes or blasts expressing GLUT1, and one reference value for the proportion of plasma cells, lymphocytes or blasts expressing ASCT2), and
c) predicting whether the subject will achieve a response with a modulator as described herein based on the correlation of the proportions determined in step a) with the reference values,
d) treating the subject with a modulator as described herein if said subject has been predicted to achieve a response with said modulator.

The method as described herein may comprise the steps of:
a) determining i) the proportion of plasma cells, lymphocytes or blasts expressing GLUT1, or ii) the proportion of plasma cells, lymphocytes or blasts expressing ASCT2, among total plasma cells, lymphocytes or blasts in a sample obtained from the subject,
b) comparing the proportion determined in step a) with a reference value,
c) predicting whether the subject will achieve a response with a modulator as described herein based on the correlation of the proportion determined in step a) with the reference value, and
d) treating the subject with a treatment other than a modulator as described herein if said subject has been predicted not to achieve a response with said modulator.

The method as described herein may comprise the steps of:
a) determining the proportion of plasma cells, lymphocytes or blasts expressing GLUT1, and the proportion of plasma cells, lymphocytes or blasts expressing ASCT2, among total plasma cells, lymphocytes or blasts in a sample obtained from the subject,
b) comparing the proportions determined in step a) with two reference values for GLUT1 and ASCT2 *(i.e.* one reference value for the proportion of plasma cells, lymphocytes or blasts expressing GLUT1, and one reference value for the proportion of plasma cells, lymphocytes or blasts expressing ASCT2), and
c) predicting whether the subject will achieve a response with a modulator as described herein based on the correlation of the proportions determined in step a) with the reference values,
d) treating the subject with a treatment other than a modulator as described herein if said subject has been predicted not to achieve a response with said modulator.

The embodiments described herein for the method to determine whether a subject suffering from a hematologic disorder will achieve or is achieving a response with a modulator as described herein apply *mutatis mutandis* to the method of treatment as described herein.

Examples of modulators, *i.e.* inhibitors of anti-apoptotic BCL-2 family proteins or agents involved in the control of phosphorylation or stability of the BCL-2 family proteins inhibitors, are described hereinabove.

The inhibitor as described herein may be an inhibitor of BCL-2, in particular, venetoclax.

Examples of treatments that may be used if the subject has been predicted not to achieve a response with modulators as described herein include, without limitation, tyrosine kinase inhibitors such as zanubrutinib, acalabrutinib, ibrutinib, or bosutinib; multi kinase inhibitors such as fedratinib, gilteritinib, midostaurin, ponatinib, or Ruxolitinib; phosphatidylinositol 3-kinase (PI3K) inhibitors such as idelalisib, copanlisib, or duvelisib; enzyme inhibitors including histone deacetylase inhibitors such as panobinostat, or belinostat, isocitrate dehydrogenase inhibitors such as ivosidenib, or enasidenib, enhancer of zeste homolog 2 (EZH2) inhibitors such as tazemetostat, and DNA and RNA methyltransferases inhibitors such as azacitidine; smoothed receptor (SMO) antagonists such as glasdegib, vismodegib or sonidegib; exportin-1 (XPO1) inhibitors such as Selinexor; proteasome inhibitors such as ixazomib citrate or carfilzomib; tubulin inhibitors such as vincristine sulfate; protein translation inhibitors such as omacetaxine mepesuccinate; purine antagonists such as mercaptopurine, immunomodulators such as pomalidomide; combinations of drugs such as the combination of decitabine and cedazuridine or the combination of cytarabine and daunorubicin; monoclonal antibodies targeting the following targets: CD19 (e.g. tafasitamab), CD20 (e.g. rituximab, obinutuzumab), CD38 (e.g. daratumumab, isatuximab), SLAMPF7 (e.g. elotuzumab), PD-1 (e.g. nivolumab, pembrolizumab), or CCR4 (e.g. mogamulizumab); bispecific antibodies such as blinatumomab; antibody-drug conjugates (ADC) such as anti-CD22 ADC (e.g. inotuzumab ozogamicin), anti-CD30 ADC (e.g. brentuximab vedotin), anti-CD79b ADC (e.g. polatuzumab vedotin), or anti-CD79b ADC (e.g. belantamab mafodotin); recombinant immunotoxins such as moxetumomab pasudotox; enzymes such as asparaginase or calaspargase; and chimeric antigen receptor T (CAR-T) cells such as tisagenlecleucel, axicabtagene ciloleucel or brexucabtagene autoleucel (see Sochacka-Cwikla et al., Cancers (Basel). 2021 Dec 24; 14(1):87, which is incorporated herein by reference).

The sample may be a bone marrow sample, a lymph node sample or a blood sample.

The sample may be a bone marrow sample. As used herein, "bone marrow sample" means any bone marrow sample derived or obtained from a subject. Collections of bone marrow samples can be performed by methods well known to those skilled in the art, including bone marrow biopsy and bone marrow aspiration.

The sample may be a lymph node sample. As used herein, "lymph node sample" means any lymph node sample derived or obtained from a subject. Collections of lymph node samples can be performed by methods well known to those skilled in the art, including lymph node biopsy.

The sample may be a blood sample. As used herein, "blood sample" means any blood sample derived or obtained from a subject. Collections of blood samples can be performed by methods well known to those skilled in the art, including venous blood sampling by venipuncture.

The present invention also relates to a kit comprising i) means for detecting GLUT1, or ii) means for detecting ASCT2, or iii) means for detecting GLUT1 and ASCT2.

According to one embodiment of the present invention, the kit comprises means for detecting GLUT1, and optionally, means for detecting ASCT2.

The kit as described herein may allow to measure the expression levels of GLUT 1 and/or ASCT2.

The expression level of GLUT1 and/or ASCT2 may be assessed at the RNA level, and the kit as described herein may comprise means for total RNA extraction, means for reverse transcription of total RNA, and means for quantifying the expression of RNA of GLUT1 and/or ASCT2. The means for quantifying the expression of RNA of GLUT1 and/or ASCT2 may be PCR or qPCR primers specific for said receptors.

The kit may also comprise reagents for carrying out a quantitative PCR (such as, for example, buffers, enzyme, and the like). The kit as described herein may also comprise means for detecting the expression level of at least one normalization gene at the RNA level.

The expression level of GLUT1 and/or ASCT2 may be assessed at the protein level, and the kit as described herein may comprise means for detecting GLUT1 and/or ASCT2.

Said means for detecting GLUT1 and/or ASCT2 may be antibodies specific for GLUT1 and ASCT2, respectively. Said means for detecting GLUT1 and/or ASCT2 may be RBD as described herein and specific for GLUT1 and ASCT2, respectively. The kit as described herein may also comprise means for detecting the expression level of at least one normalization protein or of a protein allowing monitoring of protein expression.

Examples of protein allowing monitoring protein expression include but are not limited to: MHC class I and CD326 (also known as human epithelial antigen (HEA), epithelial cell adhesion molecule (EpCAM), or epithelial-specific antigen (ESA)), transporters (from the slc family).

The kit of the invention may comprise at least one ligand, in particular a RBD ligand, as described herein coupled with at least one detecting agent as described herein.

The kit may be used to implement one of the methods as described herein. Thus, the present invention also relates to one of the methods as described herein, wherein the proportion of proportion of plasma cells, lymphocytes or blasts expressing GLUT1, and/or the proportion of proportion of plasma cells, lymphocytes or blasts expressing ASCT2, determined in a sample obtained from the subject is measured with a kit as described herein.

The present invention also relates to a computer system for stratifying prognosis of subjects suffering from a hematologic disorder as described herein. The present invention also relates to a computer-implemented method for stratifying prognosis of subjects suffering from a hematologic disorder as described herein.

The present invention also relates to a computer system for determining a personalized course of treatment of a subject suffering from a hematologic disorder as described herein. The present invention also relates to a computer-implemented method for determining a personalized course of treatment in a subject suffering from a hematologic disorder as described herein.

As used herein, the term "computer system" refers to any and all devices capable of storing and processing information and/or capable of using the stored information to control the behavior or execution of the device itself, regardless of whether such devices are electronic, mechanical, logical, or virtual in nature. The term "computer system" can refer to a single computer, but also to a plurality of computers working together to perform the function described as being performed on or by a computer system. A method implemented using a computer system is referred to as a "computer-implemented method".

The computer system according to the present invention may comprise:
- at least one processor, and
- at least one computer-readable storage medium that stores code readable by the processor.

As used herein, the term "processor" is meant to include any integrated circuit or other electronic device capable of performing an operation on at least one instruction word, such as, e.g., executing instructions, codes, computer programs, and scripts which it accesses from a storage medium. However, the term "processor" should not be construed to be restricted to hardware capable of executing software, and refers in a general way to a processing device, which can for example include a computer, a microprocessor, an integrated circuit, or a programmable logic device (PLD). The processor may also encompass one or more graphics processing units (GPU), whether exploited for computer graphics and image processing or other functions. Additionally, the instructions and/or data enabling to perform associated and/or resulting functionalities may be stored on any processor-readable medium, including, but not limited to, an integrated circuit, a hard disk, a magnetic tape (including floppy disk and zip diskette), an optical disc (including Blu-ray, compact disc and digital versatile disc), a flash memory (including memory card and USB flash drive) a random-access memory (RAM) (including dynamic and static RAM), a read-only memory (ROM) or a cache. Instructions may be in particular stored in hardware, software, firmware or in any combination thereof.

Examples of processors include, but are not limited to, central processing units (CPU), microprocessors, digital signal processors (DSPs), general purpose microprocessors, application specific integrated circuits (ASICs), field programmable logic arrays (FPGAs), and other equivalent integrated or discrete logic circuitry.

The present invention also relates to a computer program comprising software code readable by the processor adapted to perform, when executed by said processor, the computer-implemented methods as described herein.

The present invention also relates to a computer-readable storage medium comprising code readable by the processor which, when executed by said processor, causes the processor to carry out the steps of the computer-implemented methods as described herein.

Examples of computer-readable storage medium include, but are not limited to, an integrated circuit, a hard disk, a magnetic tape (including floppy disk and zip diskette), an optical disc (including Blu-ray, compact disc and digital versatile disc), a flash memory (including memory card and USB flash drive) a random-access memory (RAM) (including dynamic and static RAM), a read-only memory (ROM) or a cache.

The computer-readable storage medium may be a non-transitory computer-readable storage medium.

The code stored on the computer-readable storage medium, when executed by the processor of the computer system, may cause the processor to:
- receive an input i) proportion of plasma cells, lymphocytes or blasts expressing GLUT1, or ii) the proportion of plasma cells, lymphocytes or blasts expressing ASCT2 or iii) both proportions, among total plasma cells, lymphocytes or blasts, determined in a sample previously obtained from the subject,
- analyze and transform the input proportion(s) by organizing and/or modifying each input to derive a probability score and/or a classification label via at least one machine learning algorithm,
- generate an output, wherein the output is the classification label and/or the probability score, and
- provide a prognosis based on the output; or
- provide a personalized course or information to determine a personalized course of treatment for the subject based on the output.

The code stored on the computer-readable storage medium, when executed by the processor of the computer system, may cause the processor to:
- receive an input i) proportion of plasma cells, lymphocytes or blasts expressing GLUT1, or ii) the proportion of plasma cells, lymphocytes or blasts expressing ASCT2 or iii) both proportions, among total plasma cells, lymphocytes or blasts, determined in a sample previously obtained from the subject,
- analyze and transform the input proportion(s) by organizing and/or modifying each input level to derive a probability score and/or a classification label via at least one machine learning algorithm,
- generate an output, wherein the output is the classification label and/or the probability score, and
- provide a prognosis based on the output; or
- provide a personalized course or information to determine a personalized course of treatment for the subject based on the output.

As used herein, the terms "learning algorithm" or "machine learning algorithm" refer to computer-executed algorithms that automate analytical model building, e.g., for clustering, classification or profile recognition. Learning algorithms perform analyses on training datasets provided to the algorithm. Learning algorithms output a "model", also referred to as a "classifier", "classification algorithm" or "diagnostic algorithm". Models receive, as input, test data and produce, as output, an inference or a classification of the input data as belonging to one or another class, cluster group or position on a scale, such as diagnosis, stage, prognosis, disease progression, responsiveness to a drug, etc.

"Datasets" are collections of data used to build a machine learning mathematical model, so as to make data-driven predictions or decisions. In "supervised learning" (i.e., inferring functions from known input-output examples in the form of labelled training data), three types of machine learning datasets are typically dedicated to three respective kinds of tasks: "training", i.e., fitting the parameters; "validation", i.e., tuning machine learning hyperparameters (which are parameters used to control the learning process); and "testing", i.e., checking independently of a training dataset exploited for building a mathematical model that the latter model provides satisfying results.

A variety of learning algorithms can be used to infer a condition or state of a subject. Machine learning algorithms may be supervised or unsupervised. Learning algorithms include, but are not limited to, artificial neural networks (e.g., back propagation networks), discriminant analyses (e.g., Bayesian classifier, Fischer analysis), support vector machines, decision trees (e.g., recursive partitioning processes, such as classification and regression trees [CART]), random forests, linear classifiers
- (e.g., multiple linear regression [MLR], partial least squares [PLS] regression, principal components regression [PCR]), hierarchical clustering and cluster analysis. The learning algorithm generates a model or classifier that can be used to make an inference,
- e.g., an inference about a disease state of a subject.

The at least one machine learning algorithm may be previously trained with at least one training dataset.

The at least one training dataset may comprise information relating to i) the proportion of plasma cells, lymphocytes or blasts expressing GLUT1, or ii) the proportion of plasma cells, lymphocytes or blasts expressing ASCT2 or iii) both proportions, among total plasma cells, lymphocytes or blasts, from samples previously obtained from reference subjects.

The at least one machine learning algorithm may be selected from the group comprising or consisting of an artificial neural network (ANN), a perceptron algorithm, a deep neural network, a clustering algorithm, a k-nearest neighbors algorithm (k-NN), a decision tree algorithm, a random forest algorithm, a linear regression algorithm, a logistic regression algorithm, a linear discriminant analysis (LDA) algorithm, a quadratic discriminant analysis (QDA) algorithm, a support vector machine (SVM), a Bayes algorithm, a simple rule algorithm, a clustering algorithm, a meta-classifier algorithm, a Gaussian mixture model (GMM) algorithm, a nearest centroid algorithm, a gradient boosting algorithm (such as, e.g., an extreme gradient boosting [XG Boost] algorithm or an adaptative boosting [AdaBoost] algorithm), a linear mixed effects model algorithm, and a combination thereof.

### TABLE OF SEQUENCES

The sequences listed in the present application are presented in the following table.

| SEQ ID NO | Sequence |
|---|---|
| 1 | tct ccc age gtg cgc cat |
| 2 | MALPYHIFLFTVVSPSFTLTAPPPCRCMTSSSPYQEFLWRMQRPGNIDAPS YRSLCKGTPTFTAHTHMPRNCYHSATLCMHANTHYWTGKMINPSCPGGL GVTVCWTYFTQTGMSDGGGVQDQAREKHVKEVISQLTRVHGTSSPYKG LD LSKLHETLRT HTRLVSLFNT TLTGLHEVSA QNPTNCWIC |
| 3 | MALPYHIFLFTVLLPSFTLTAPPPCRCMTSSSPYQEFLWRMQRPGNIDAPS YRSLCKGTPTFTAHTHMPRNCYHSATLCMHANTHYWTGKMINPSCPGGL GVTVCWTYFTQTGMSDGGGVQDQAREKHVKEVISQLTRVHGTSSPYKG LDLSKLHETLRTHTRLVSLFNTTLTGLHEVSAQNPTNCWIC |
| 4 | MALPYHIFLFTVLLPSFTLTAPPPCRCMTSSSPYQEFLWRMQRPGNIDAPS YRSLSKGTPTFTAHTHMPRNCYHSATLCMHANTHYWTGKMINPSCPGGL GVTVCWTYFTQTGMSDGGGVQ |
| 5 | MALPYHIFLFTVLLPSFTLTAPPPCRCMTSSSPYQEFLWRMQRPGNIDAPS YRSLSKGTPTFTAHTHMPRNCYHSATLCMHANTHYWTGKMINPSCPGGL GVTVCWTYFTQTGMSDGGGVQDQAREKHVKEVISQLTRVHGTSSPYKG LDLSKLHETLRTHTRLVSLFNTTLTGLHEVSAQ |
| 6 | MKLPTGMVILCSLIIVRAGFDDPRKAIALVQKQHGKPCECSGGQVSEAPPN SIQQVTCPGKTAYLMTNQKWKCRVTPKISPSGGELQNCPCNTFQDSMHSS CYTEYRQCRRINKTYYTATLLKIRSGSLNEVQILQNPNQLLQSPCRGSINQP VCWSATAPIHISDGGGPLDTKRVWTVQKRLEQIHKAMTPELQYHPLALPK VRDDLSLDARTFDILNTTFRLLQMSNFSLAQDCWLC |
| 7 | MKLPTGMVILCSLIIVRAGFDDPRKAIALVQKQHGKPCECSGGQVSEAPPN SIQQVTCPGKTAYLMTNQKWKCRVTPKISPSGGELQNCPCNTFQDSMHSS |
| | CYTEYRQCRRINKTYYTATLLKIRSGSLNEVQILQNPNQLLQSPCRGSINQP VCWSATAPIHISDGGGPLDTKRVWTVQKRLEQIHKAMTPELQYHPLALPK VRDDLSLDARTFDILNTTF |
| 8 | MKLPAGMVILCSLIIVRAGFDDPRKAIALVQKQHGKPCECSGGQVSEAPP NSIQQVTCPGKTAYLMTNQKWKCRVTPKNLTPSGGELQNCPCNTFQDSM HSSCYTEYRQCRANNKTYYTATLLKIRSGSLNEVQILQNPNQLLQSPCRGS INQPVCWSATAPIHISDGGGPLDTKRVWTVQKRLEQIHKAMHPELQYHPL ALPKVRDDLSLDARTFDILNTTF |
| 9 | MKPPAGMVFLWVLTSLGAGIGAKIVKEGNPHQVYTLTWQIYSQSGEVVW EVQGNHALNTWWPPLTPDFCQLAAGLDTWDIPARSPKNLQSYMGERIQQ MTAHGCSSPTARCRLAQAEFYVCPRDNRDRATAHRCGGYEEYFCSAWG CETTGDAYWQPTSSWDLITITRGYTKPDPDGHTCYYKKGTEGYHHWISPL SLPLKITFTDSGKRALGWQTGYTWGLRWYLPGKDRGIVLKIKLKIDTITQT VGPNLVLADQKAPVQLAIPVQPPRAPTQTPGINPVNSTLSPSLGYPTPPLDR AQGDRLLNLVQGVYLTLNLTAPNQTQDCWLCL |
| 10 | MKLPTGMVILCSLIIVRAGFDDPRKAIALVQKQHGKPCECSGGQVSEAPPN SIQQVTCPGKTAYLMTNQKWKCRVTPKISPSGGELQNCPCNTFQDSMHSS CYTEYRQCRRINKTYYTATLLKIRSGSLNEVQILQNPNQLLQSPCRGSINQP VCWSATAPIHISDGGGPLDTKRVWTVQKRLEQIHKAMTPELQYHPLALPK VRDDLSLDARTFDILNTTF |
| 11 | MGFTTKIIFLYNLVLVYAGFDDPRKAIELVQKRYGRPCDCSGGQVSEPPSD RVSQVTCSGKTAYLMPDQRWKCKSIPKDTSPSGPLQECPCNSYQSSVHSS CYTSYQQCRSGNKTYYTATLLKTQTGGTSDVQVLGSTNKLIQSPCNGIKG QSICWSTTAPIHVSDGGGPLDTTRIKSVQRKLEEIHKALYPELQYHPLAIPK VRDNLMVDAQTLNILNATYNLLLMSNTSLVDDCWLC |
| 12 | MDCLTNLRSAEGKVDQASKILILLVAWWGFGTTAEGYPLQQLWELPCDC SGGYVSSIPTYYTYSLDCGGSTAYLTYGSGTGSWSWGGGFKQQWECVFK PKIIPSVQGQPGPCPSECLQIATQMHSTCYEKTQECTLLGKTYFTAILQKTK LGSYEDGPNKLIQASCTGTVGKPVCWDPVAPVYYVSDGGGPTDMIREESV RERLEEIIRHSYPSVQYHPLALPRSRGVDLDPQTSDILEATHQVLNATNPQL AENCWLC |
| 13 | MDCLTNLRSAEGKVDQASKILILLVAWWGFGTTAEGYPLQQLWELPCDC SGGYVSSIPTYYTYSLDCGGSTAYLTYGSGTGSWSWGGGFKQQWECVFK PKIIPSVQGQPGPCPSECLQIATQMHSTCYEKTQECTLLGKTYFTAILQKTK LGSYEDGPNKLIQASCTGTVGKPVCWDPVAPVYVSDGGGPTDMIREESV RERLEEIIRHSYPSVQYHPLALPRSRGVDLDPQTSDILEATHQVLNATNPK LAENCWLCMTLGTPIPAAIPTNGNVTLDGNCSLSLPFGCNPPGSIDVSCYA GEADNRTGIPVGYVHFTNCTSIQEVTNETSQMGNLTRLCPPPGHVFVCGN NMAYTALPNKWIGLCILASIVPDISIISGEEPIPLPSIEYTARRHKRAVQFIPL LVGLGISGATLAGGTGLGVSVHTYHKLSNQLIEDVQALSGTINDLQDQIDS LAEVVLQNRRGLDLLTAEQGGICLALQEKCCFYANKSGIVRDKIRKLQED LIERKRALYDNPLWSGLNGFLPYLLPLLGPLFGLILFLTLGPCIMKTLTRIIH DKIQAVKS |
| 14 | MNFNHHFTWSLVIISQIFQVQAGFGDPREALLEIQQKHGKPCDCAGGYVS SPPTNSLTTVSCSTYTAYSVTNSLKWQCVSTPTTASPTHIGSCPSQCNSQSY DSVHATCYNHYQQCTIGNKTYLTATMIRDKSPSSGDGNVPTILGNNQNLII |
| | AGCPENKKGQVVCWNSQPSVHMSDGGGPQDKVREIIVNKKFEELHKSLF PELSYHPLALPEARGKEKIDAHTFDLLATVHSLLNVSSQRQLAEDCWLC |
| 15 | MNFNYHFIWSLVILSQISQVQAGFGDPREALAEIQQKHGKPCDCAGGYVS SPPINSLTTVSCSTHTAYSVTNSLKWQCVSTPTTPSNTHIGSCPGECNTISY DSVHASCYNHYQQCNIGNKTYLTATITGDRTPAIGDGNVPTVLGTSHNLIT AGCPNGKKGQVVCWNSRPSVHISDGGGPQDKARDIIVNKKFEELHRSLFP ELSYHPLALPEARGKEKIDAHTLDLLATVHSLLNASQPSLAEDCWLC |
| 16 | MGKFLATLILFFQFCPLILGDYSPSCCTLTIGVSSYHSKPCNPAQPVCSWTL DLLALSADQALQPPCPNLVSYSSYHATYSLYLFPHWIKKPNRNGGGYYSA SYSDPCSLKCPYLGCQSWTCPYTGAVSSPYWKFQQDVNFTQEVSRLNINL HFSKCGFPFSLLVDAPGYDPIWFLNTEPSQLPPTAPPLLPHSNLDHILEPSIP WKSKLLTLVQLTLQSTNYTCIVCIDRASLSTWHVLYSPNVSVPSSSSTPLL YPSLALPAPHLTLPFNWTHCFDPQIQAIVSSPCHNSLILPPFSLSPVPTLGSR SRR |
| 17 | DYSPSCCTLTIGVSSYHSKPCNPAQPVCSWTLDLLALSADQALQPPCPNLV SYSSYHATYSLYLFPHWIKKPNRNGGGYYSASYSDPCSLKCPYLGCQSWT CPYTGAVSSPYWKFQQDVNFTQEVSRLNINLHFSKCGFPFSLLVDAPGYD PIWFLNTEPSQLPPTAPPLLPHSNLDHILEPSIPWKSKLLTLVQLTLQSTNYT CIVCIDRASLSTWHVLYSPNVSVPSSSSTPLLYPSLALPAPHLTLPFNWTHC FDPQIQAIVSSPCHNSLILPPFSLSPVPTLGSRSRR |
| 18 | MGKFLATLILFFQFCPLIFGDYSPSCCTLTIGVSSYHSKPCNPAQPVCSWTL DLLALSADQALQPPCPNLVSYSSYHATYSLYLFPHWTKKPNRNGGGYYS ASYSDPCSLKCPYLGCQSWTCPYTGAVSSPYWKFQHDVNFTQEVSRLNIN LHFSKCGFPFSLLVDAPGYDPIWFLNTEPSQLPPTAPPLLPHSNLDHILEPSI PWKSKLLTLVQLTLQSTNYTCIVC |
| 19 | MGKFLATLILFFQFCPLIFGDYSPSCCTLTIGVSSYHSKPCNPAQPVCSWTL DLLALSADQALQPPCPNLVSYSSYHATYSLYLFPHWTKKPNRNGGGYYS ASYSDPCSLKCPYLGCQSWTCPYTGAVSSPYWKFQHDVNFTQEVSRLNIN LHFSKCGFPFSLLVDAPGYDPIWFLNTEPSQ |
| 20 | IKKPNPNGGGYYLASYSDPCSLKCPYLGCQSWTCPYTGAVSSPYWKFQQ DV |
| 21 | VKKPNRNGGGYYLASYSDPCSLKCPYLGCQSWTCPYTGAVSSPYWKFQQ DV |
| 22 | IKKPNRNGGGYYLASYSDPCSLKCPYLGCQSWTCPYTGAVSSPYWKFQQ DV |
| 23 | IKKPNRNGGGYYLASYSDPCSLKCPYLGCQSWTCPYTGPVSSPYWKFQQ DV |
| 24 | IKKPNRNGGGYHSASYSDPCSLKCPYLGCQSWTCPYAGAVSSPYWKFQQ DVNFTQEV |
| 25 | DYSPSCCTLTIGVSSYHSKPCNPAQPVCSWTLDLLALSADQALQPPCPNLV SYSSYHATYSLYLFPHWTKKPNRNGGGYYSASYSDPCSLKCPYLGCQSW TCPYTGAVSSPYWKFQHDVNFTQEVSRLNINLHFSKCGFPFSLLVDAPGY DPIWFLNTEPSQLPPTAPPLLPHSNLDHILEPSIPWKSKLLTLVQLTLQSTNY TCIVC |
| 26 | DYSPSCCTLTIGVSSYHSKPCNPAQPVCSWTLDLLALSADQALQPPCPNLV SYSSYHATYSLYLFPHWTKKPNRNGGGYYSASYSDPCSLKCPYLGCQSW |
| | TCPYTGAVSSPYWKFQHDVNFTQEVSRLNINLHFSKCGFPFSLLVDAPGY DPIWFLNTEPSQ |
| 27 | MGNVFFLLLFSLTHFPPVQQSRCTLTVGISSYHSSPCSPTQPVCTWNLDLN SLTTDQRLHPPCPNLITYSGFHKTYSLYLFPHWIKKPNRQGLGYYSPSYND PCSLQCPYLGCQSWTCPYTGPVSSPSWKFHSDVNFTQEVSQVSLRLHFSK CGSSMTLLVDAPGYDPLWFITSEPTQPPPTPPPLVHDSDLEHVLTPSTSWT TKMLKFIQLTLQSTNYSCMVCVDRSSLSSWHVLYTPNISIPQQTSSRTILFP SLALPAPPFQPFPWTHCYQPRLQAITTDDCNNSIILPPFSLAPVPPPATRRRR |
| 28 | SRCTLTVGISSYHSSPCSPTQPVCTWNLDLNSLTTDQRLHPPCPNLITYSGF HKTYSLYLFPHWIKKPNRQGLGYYSPSYNDPCSLQCPYLGCQSWTCPYTG PVSSPSWKFHSDVNFTQEVSQVSLRLHFSKCGSSMTLLVDAPGYDPLWFI TSEPTQPPPTPPPLVHDSDLEHVLTPSTSWTTKMLKFIQLTLQSTNYSCMV CVDRSSLSSWHVLYTPNISIPQQTSSRTILFPSLALPAPPFQPFPWTHCYQPR LQAITTDDCNNSIILPPFSLAPVPPPATRRRR |
| 29 | MGNVFFLLLFSLTHFPLAQQSRCTLTVGISSYHSSPCSPTQPVCTWNLDLN SLTTDQRLHPPCPNLITYSGFHKTYSLYLFPHWIKKPNRQGLGYYSPSYND PCSLQCPYLGCQSWTCPYTGPVSSPSWKFHSDVNFTQEVSQVSLRLHFSK CGSSMTLLVDAPGYDPLWFITSEPTQPPPTSPPLVHDSDLEHVLTPSTSWT TKILKFIQLTLQSTNYSCMVC |
| 30 | MGNVFFLLLFSLTHFPLAQQSRCTLTVGISSYHSSPCSPTQPVCTWNLDLN SLTTDQRLHPPCPNLITYSGFHKTYSLYLFPHWIKKPNRQGLGYYSPSYND PCSLQCPYLGCQSWTCPYTGPVSSPSWKFHSDVNFTQEVSQVSLRLHFSK CGSSMTLLVDAPGYDPLWFITSEPTQ |
| 31 | IRKPNRQGLGYYSPSYNDPCSLQCPYLGSQSWTCPYTAPVSTPSWNFHSD V |
| 32 | SRCTLTVGISSYHSSPCSPTQPVCTWNLDLNSLTTDQRLHPPCPNLITYSGF HKTYSLYLFPHWIKKPNRQGLGYYSPSYNDPCSLQCPYLGCQSWTCPYTG PVSSPSWKFHSDVNFTQEVSQVSLRLHFSKCGSSMTLLVDAPGYDPLWFI TSEPTQPPPTSPPLVHDSDLEHVLTPSTSWTTKILKFIQLTLQSTNYSCMVC |
| 33 | SRCTLTVGISSYHSSPCSPTQPVCTWNLDLNSLTTDQRLHPPCPNLITYSGF HKTYSLYLFPHWIKKPNRQGLGYYSPSYNDPCSLQCPYLGCQSWTCPYTG PVSSPSWKFHSDVNFTQEVSQVSLRLHFSKCGSSMTLLVDAPGYDPLWFI TSEPTQ |
| 34 | MGKSGLYFSLICFYTLFPSSFGNPSRCTLFIGASSYHSDPCGSNHPRCTWRL DLFSLTKDQSLSPPCPGLVTYSQYHKPYSLYVFPHWIAKPDRRGLGYYSA SYSDPCAIQCPYLGCQSWTCPYTGPVSNPHWKYTSDLNFTQEVSSISLHLH FSKCGSSFSFLLDAPGYDPVWLLSSQATQIPPTPAPLIQDSDLQHILEPSIPW SSKILNLILLALKSTNYSCMVCVDRSSLSSWHVLYDPLKAPSSPDPQAQSIL RPSLAIPASNITPPFPWTHCYRPPLQAISSENCNNSVILPPFSLSPIPDVSRPR KRR |
| 35 | NPSRCTLFIGASSYHSDPCGSNHPRCTWRLDLFSLTKDQSLSPPCPGLVTYS QYHKPYSLYVFPHWIAKPDRRGLGYYSASYSDPCAIQCPYLGCQSWTCPY TGPVSNPHWKYTSDLNFTQEVSSISLHLHFSKCGSSFSFLLDAPGYDPVWL LSSQATQIPPTPAPLIQDSDLQHILEPSIPWSSKILNLILLALKSTNYSCMVC VDRSSLSSWHVLYDPLKAPSSPDPQAQSILRPSLAIPASNITPPFPWTHCYR PPLQAISSENCNNSVILPPFSLSPIPDVSRPRKRR |
| 36 | MGKSGLYFSLICFYTLFPSSFGNPSRCTLFIGASSYHSDPCGSNHPRCTWRL DLFSLTKDQSLSPPCPGLVTYSQYHKPYSLYVFPHWIAKPDRRGLGYYSA SYSDPCAIQCPYLGCQSWTCPYTGPVSNPHWKYTSDLNFTQEVSSISLHLH FSKCGSSFSFLLDAPGYDPVWLLSSQA |
| 37 | NPSRCTLFIGASSYHSDPCGSNHPRCTWRLDLFSLTKDQSLSPPCPGLVTYS QYHKPYSLYVFPHWIAKPDRRGLGYYSASYSDPCAIQCPYLGCQSWTCPY TGPVSNPHWKYTSDLNFTQEVSSISLHLHFSKCGSSFSFLLDAPGYDPVWL LSSQA |
| 38 | MGNVLFLTLLATLGIPVLQASRCTITVGISSYHSSPCSPAQPLCTWALDLVS ITKDQLLYPPCQNLITYSNYHKTYSLYLFPHWVQKPLRRGLGYYSASYSD PCSLQCPYLGSQSWTCPYTGPVSSPTWRFSTDVNFTQEVSRVSLKLHFSKC GSSLTLLIDAPGYDPLWYLTSEPTQEPPTPPPLVSDSDLEHVLTPSASWASK MLTLIHLTLQSTNYSCMVCIDRASLSSWHVLYTPNISSNAPSKPIVRPSLAL SAPRPQPFPWTHCYQPQVQAVTTAKCNNSIILPPFSLSPLPGAPLTRRRR |
| 39 | SRCTITVGISSYHSSPCSPAQPLCTWALDLVSITKDQLLYPPCQNLITYSNY HKTYSLYLFPHWVQKPLRRGLGYYSASYSDPCSLQCPYLGSQSWTCPYT GPVSSPTWRFSTDVNFTQEVSRVSLKLHFSKCGSSLTLLIDAPGYDPLWYL TSEPTQEPPTPPPLVSDSDLEHVLTPSASWASKMLTLIHLTLQSTNYSCMV CIDRASLSSWHVLYTPNISSNAPSKPIVRPSLALSAPRPQPFPWTHCYQPQV QAVTTAKCNNSIILPPFSLSPLPGAPLTRRRR |
| 40 | MGNVLFLTLLATLGIPVLQASRCTITVGISSYHSSPCSPAQPLCTWALDLVS ITKDQLLYPPCQNLITYSNYHKTYSLYLFPHWVQKPLRRGLGYYSASYSD PCSLQCPYLGSQSWTCPYTGPVSSPTWRFSTDVNFTQEVSRVSLKLHFSKC GSSLTLLIDAPGYDPLWYLTSEPTQEPPTPPPLVSDSDLEHVLTPSASWASK MLTLIHLTLQSTNYSCMVC |
| 41 | MGNVLFLTLLATLGIPVLQASRCTITVGISSYHSSPCSPAQPLCTWALDLVS ITKDQLLYPPCQNLITYSNYHKTYSLYLFPHWVQKPLRRGLGYYSASYSD PCSLQCPYLGSQSWTCPYTGPVSSPTWRFSTDVNFTQEVSRVSLKLHFSKC GSSLTLLIDAPGYDPLWYLTSEPTQ |
| 42 | SRCTITVGISSYHSSPCSPAQPLCTWALDLVSITKDQLLYPPCQNLITYSNY HKTYSLYLFPHWVQKPLRRGLGYYSASYSDPCSLQCPYLGSQSWTCPYT GPVSSPTWRFSTDVNFTQEVSRVSLKLHFSKCGSSLTLLIDAPGYDPLWYL TSEPTQEPPTPPPLVSDSDLEHVLTPSASWASKMLTLIHLTLQSTNYSCMV C |
| 43 | SRCTITVGISSYHSSPCSPAQPLCTWALDLVSITKDQLLYPPCQNLITYSNY HKTYSLYLFPHWVQKPLRRGLGYYSASYSDPCSLQCPYLGSQSWTCPYT GPVSSPTWRFSTDVNFTQEVSRVSLKLHFSKCGSSLTLLIDAPGYDPLWYL TSEPTQ |
| 44 | MGKFLATLILFFQFCPLILGDYSPSCCTLTIGVSSYLSKPCNPAQPVCSWTL DLLALSADQALQPPCPNLVSYSSYHATYSLYLFPHWIKKPNRNGGGYYSA SYSDPCSLKCPYLGCQSWTCPYTGVVSSPYWKFQQDVNFTQEVSHLNINL HFSKCGFPFSLLIDAPGYDPIWFLNTEPSQLPPTAPPLLPHSNLDHILEPSIP WKSKLLTLVQLTLQSTNYTCIVCIDRASLSTWHVLYSPNVSVPSSSSTPLL YPSLALPAPHLTLPFNWTHCFDPQIQAIVSSPCHNSLILPPFSLSPVPTLGSR SRR |
| 45 | DYSPSCCTLTIGVSSYLSKPCNPAQPVCSWTLDLLALSADQALQPPCPNLV SYSSYHATYSLYLFPHWIKKPNRNGGGYYSASYSDPCSLKCPYLGCQSWT |
| | CPYTGVVSSPYWKFQQDVNFTQEVSHLNINLHFSKCGFPFSLLIDAPGYDP IWFLNTEPSQLPPTAPPLLPHSNLDHILEPSIPWKSKLLTLVQLTLQSTNYTC IVCIDRASLSTWHVLYSPNVSVPSSSSTPLLYPSLALPAPHLTLPFNWTHCF DPQIQAIVSSPCHNSLILPPFSLSPVPTLGSRSRR |
| 46 | MGKFLATLILFFQFCPLILGDYSPSCCILTIGVSSYHSKPCNPAQPVCSWTL DLLALSADQALQPPCPNLVSYSSYHATYSLYLFPHWIKKPNRNGGGYYSA SYSDPCSLKCPYLGCQSWTCPYTGAVSSPYWKFQQDVNFTQEVSHLNINL HFSKCGFPFSLLIDAPGYDPIWFLNTE |
| 47 | DYSPSCCILTIGVSSYHSKPCNPAQPVCSWTLDLLALSADQALQPPCPNLV SYSSYHATYSLYLFPHWIKKPNRNGGGYYSASYSDPCSLKCPYLGCQSWT CPYTGAVSSPYWKFQQDVNFTQEVSHLNINLHFSKCGFPFSLLIDAPGYDP IWFLNTE |
| 48 | MGKIIAFLLFHLTCITITKQSRCTLTVGVSSYHSSPCSLAQPICTWDLDLHSL TTDQRLYPPCPNLVSYSNFHKSYSLYLFPHWVKKPNRQGLGYYSASYSDP CSLQCPYLGSQSWTCPYTGPISSPSWRFHRDVNFTQEVNHVTLRLHFSRC GSSMTLLIDAPGYDPLWFISSEPTQPPPTSPPLVRDSDLEHILTPSSSWATR MLTLIQLTLQSTNYSCMVCIDRTSLSSWHVLYTPNISASPGGDSLPILYPSL ALPAPQPQPFSWSHCYQPHLQAVTTANCNNSIVLPPFSLTPVPSPGTRSRR |
| 49 | SRCTLTVGVSSYHSSPCSLAQPICTWDLDLHSLTTDQRLYPPCPNLVSYSN FHKSYSLYLFPHWVKKPNRQGLGYYSASYSDPCSLQCPYLGSQSWTCPYT GPISSPSWRFHRDVNFTQEVNHVTLRLHFSRCGSSMTLLIDAPGYDPLWFI SSEPTQPPPTSPPLVRDSDLEHILTPSSSWATRMLTLIQLTLQSTNYSCMVCI DRTSLSSWHVLYTPNISASPGGDSLPILYPSLALPAPQPQPFSWSHCYQPHL QAVTTANCNNSIVLPPFSLTPVPSPGTRSRR |
| 50 | MGKIIAFLLFHLTCITITKQSRCTLTVGVSSYHSSPCSLAQPICTWDLDLHSL TTDQRLYPPCPNLVSYSNFHKSYSLYLFPHWVKKPNRQGLGYYSASYSDP CSLQCPYLGSQSWTCPYTGPISSPSWRFHRDVNFTQEVNHVTLRLHFSRC GSSMTLLIDAPGYDPLWFISSE |
| 51 | SRCTLTVGVSSYHSSPCSLAQPICTWDLDLHSLTTDQRLYPPCPNLVSYSN FHKSYSLYLFPHWVKKPNRQGLGYYSASYSDPCSLQCPYLGSQSWTCPYT GPISSPSWRFHRDVNFTQEVNHVTLRLHFSRCGSSMTLLIDAPGYDPLWFI SSE |
| 52 | MGKFGLYCLVHLYILLPASSGNPSRCTLFIGASSYHSSPCGSSLPRCTWNL DLFSLTKDQSLSPPCPDLITYSQYHKPYSLYVFPHWITKPNRRGLGYYSAS YSDPCAIQCPYLGCQSWTCPYTGPVSSPHWRYTYDLNFTQEVSSVSLHLH FSKCGSSFSFLLDAPGYDPVWFLSSQATQAPPTPAPLIRDSDLQYILEPPIP WSSKILNLILLTLKSTNYSCMVCVDRSSLSSWHVLYGPTQVPSPPDPQARS ILRPALAIPASNITPPFPWTHCYRPPPQAISSENCNNSVVLPPFSLSPIPNVSR PRKRR |
| 53 | NPSRCTLFIGASSYHSSPCGSSLPRCTWNLDLFSLTKDQSLSPPCPDLITYSQ YHKPYSLYVFPHWITKPNRRGLGYYSASYSDPCAIQCPYLGCQSWTCPYT GPVSSPHWRYTYDLNFTQEVSSVSLHLHFSKCGSSFSFLLDAPGYDPVWF LSSQATQAPPTPAPLIRDSDLQYILEPPIPWSSKILNLILLTLKSTNYSCMVC VDRSSLSSWHVLYGPTQVPSPPDPQARSILRPALAIPASNITPPFPWTHCYR PPPQAISSENCNNSVVLPPFSLSPIPNVSRPRKRR |
| 54 | MGKSGFYFCFIYTLFPASFGNPSRCTLFIGASSYHSDPCGSNHPQCTWRLD LFSLTRDQSLSPPCPDLVTYSQYHKPYSLYVFPHWMAKPNRQGLGYYSAS |
| | YSDPCAIQCPYLGCQSWTCPYTGPVSSPHWKYSSDLNFTQEVSSISLHLHF SKCGSSFSFLLDAPGYDPVWFLSSQATQV |
| 55 | NPSRCTLFIGASSYHSDPCGSNHPQCTWRLDLFSLTRDQSLSPPCPDLVTYS QYHKPYSLYVFPHWMAKPNRQGLGYYSASYSDPCAIQCPYLGCQSWTCP YTGPVSSPHWKYSSDLNFTQEVSSISLHLHFSKCGSSFSFLLDAPGYDPVW FLSSQATQV |
| 56 | MGKSLFFFCIIVQACLPTLCDRGPSCCTLTVGVSSYHSKPCNPTQPICSWTL DLLTLTTDQALQPPCPNLIGYSNYHATYPLYLFPHWVKKPNRGGGGYYS ASYSDPCSLKCPYLGCQSWTCPYTGAVSGPYWKYQTDVNFTQDVSRLTV HLHFSKCGFPFSLLIDAPGYDPIWLINSEPSQLPPTAPPLLPHSNLDHILEPSI PWKSKLLTLVQLTLQSTNYTCIVCLDRASLSTWHVLYSPNMSVPSSPSVPL IYPSLALPAPHLALPFNWTHCFDPKLQAITSAHCYNALILPPFSLSPVPALS HRTRR |
| 57 | CDRGPSCCTLTVGVSSYHSKPCNPTQPICSWTLDLLTLTTDQALQPPCPNLI GYSNYHATYPLYLFPHWVKKPNRGGGGYYSASYSDPCSLKCPYLGCQSW TCPYTGAVSGPYWKYQTDVNFTQDVSRLTVHLHFSKCGFPFSLLIDAPGY DPIWLINSEPSQLPPTAPPLLPHSNLDHILEPSIPWKSKLLTLVQLTLQSTNY TCIVCLDRASLSTWHVLYSPNMSVPSSPSVPLIYPSLALPAPHLALPFNWT HCFDPKLQAITSAHCYNALILPPFSLSPVPALSHRTRR |
| 58 | MGNVFFLLLFSLTHFPLAQQSRCTLTVGISSYHSSPCSPTQPVCTWNLDLN SLTTDQRLHPPCPNLITYSGFHKTYSLYLFPHWIKKPNRQGLGYYSPSYND PCSLQCPYLGCQSWTCPYTGPVSSPSWKFHSDVNFTQEVSQVSLRLHFSK CGSSMTLLVDAPGYDPLWFITSEPTQ |
| 59 | APSTCSKPTCPPPELLGGPSVFIFPPKPKDTLMISRTPEVTCVVVDVSQDDP EVQFTWYINNEQVRTARPPLREQQFDCTIRVVSTLPIAHQDWLRGKEFKC KVHNKALPAPIEKTISKARGQPLEPKVYTMGPPREELSSRSVSLTCMINGF YPSDISVEWEKNGKAEDNYKTTPAVLDSDGSYFLYSKLSVPTSEWQRGD VFTCSVMHEALHNHYTQKSISRSPGK |
| 60 | VDVPRDCGCKPCICTVPEVSSVFIFPPKPKDVLTITLTPKVTCVVVDISKDD PEVQFSWFVDDVEVHTAQTQPREEQFNSTFRSVSELPIMHQDWLNGKEFK CRVNSAAFPAPIEKTISKTKGRPKAPQVYTIPPPKEQMAKDKVSLTCMITD FFPEDITVEWQWNGQPAENYKNTQPIMDTDGSYFVYSKLNVQKSNWEAG NTFTCSVLHEGLHNHHTEKSLSHSPGK |
| 61 | MALPYHIFLFTVLLPSFTLTAPPPCRCMTSSSPYQEFLWRMQRPGNIDAPS YRSLSKGTPTFTAHTHMPRNCYHSATLCMHANTHYWTGKMINPSCPGGL GVTVCWTYFTQTGMSDGGGVQGSVDVPRDCGCKPCICTVPEVSSVFIFPP KPKDVLTITLTPKVTCVVVDISKDDPEVQFSWFVDDVEVHTAQTQPREEQ FNSTFRSVSELPIMHQDWLNGKEFKCRVNSAAFPAPIEKTISKTKGRPKAP QVYTIPPPKEQMAKDKVSLTCMITDFFPEDITVEWQWNGQPAENYKNTQ PIMDTDGSYFVYSKLNVQKSNWEAGNTFTCSVLHEGLHNHHTEKSLSHSP GK |
| 62 | MKLPTGMVILCSLIIVRAGFDDPRKAIALVQKQHGKPCECSGGQVSEAPPN SIQQVTCPGKTAYLMTNQKWKCRVTPKISPSGGELQNCPCNTFQDSMHSS CYTEYRQCRRINKTYYTATLLKIRSGSLNEVQILQNPNQLLQSPCRGSINQP VCWSATAPIHISDGGGPLDTKRVWTVQKRLEQIHKAMTPELQYHPLALPK VRDDLSLDARTFDILNTTFGSVDVPRDCGCKPCICTVPEVSSVFIFPPKPKD VLTITLTPKVTCVVVDISKDDPEVQFSWFVDDVEVHTAQTQPREEQFNSTF |
| | RSVSELPIMHQDWLNGKEFKCRVNSAAFPAPIEKTISKTKGRPKAPQVYTI PPPKEQMAKDKVSLTCMITDFFPEDITVEWQWNGQPAENYKNTQPIMDT DGSYFVYSKLNVQKSNWEAGNTFTCSVLHEGLHNHHTEKSLSHSPGK |
| 63 | MKLPAGMVILCSLIIVRAGFDDPRKAIALVQKQHGKPCECSGGQVSEAPP NSIQQVTCPGKTAYLMTNQKWKCRVTPKNLTPSGGELQNCPCNTFQDSM HSSCYTEYRQCRANNKTYYTATLLKIRSGSLNEVQILQNPNQLLQSPCRGS INQPVCWSATAPIHISDGGGPLDTKRVWTVQKRLEQIHKAMHPELQYHPL ALPKVRDDLSLDARTFDILNTTFGSVDVPRDCGCKPCICTVPEVSSVFIFPP KPKDVLTITLTPKVTCVVVDISKDDPEVQFSWFVDDVEVHTAQTQPREEQ FNSTFRSVSELPIMHQDWLNGKEFKCRVNSAAFPAPIEKTISKTKGRPKAP QVYTIPPPKEQMAKDKVSLTCMITDFFPEDITVEWQWNGQPAENYKNTQP IMDTDGSYFVYSKLNVQKSNWEAGNTFTCSVLHEGLHNHHTEKSLSHSP GK |
| 64 | MGNVFFLLLFSLTHFPLAQQSRCTLTVGISSYHSSPCSPTQPVCTWNLDLN SLTTDQRLHPPCPNLITYSGFHKTYSLYLFPHWIKKPNRQGLGYYSPSYND PCSLQCPYLGCQSWTCPYTGPVSSPSWKFHSDVNFTQEVSQVSLRLHFSK CGSSMTLLVDAPGYDPLWFITSEPTQGSAPSTCSKPTCPPPELLGGPSVFIFP PKPKDTLMISRTPEVTCVVVDVSQDDPEVQFTWYINNEQVRTARPPLREQ QFDCTIRVVSTLPIAHQDWLRGKEFKCKVHNKALPAPIEKTISKARGQPLE PKVYTMGPPREELSSRSVSLTCMINGFYPSDISVEWEKNGKAEDNYKTTP AVLDSDGSYFLYSKLSVPTSEWQRGDVFTCSVMHEALHNHYTQKSISRSP GK |
| 65 | SRCTLTVGISSYHSSPCSPTQPVCTWNLDLNSLTTDQRLHPPCPNLITYSGF HKTYSLYLFPHWIKKPNRQGLGYYSPSYNDPCSLQCPYLGCQSWTCPYTG PVSSPSWKFHSDVNFTQEVSQVSLRLHFSKCGSSMTLLVDAPGYDPLWFI TSEPTQGSAPSTCSKPTCPPPELLGGPSVFIFPPKPKDTLMISRTPEVTCVVV DVSQDDPEVQFTWYINNEQVRTARPPLREQQFDCTIRVVSTLPIAHQDWL RGKEFKCKVHNKALPAPIEKTISKARGQPLEPKVYTMGPPREELSSRSVSL TCMINGFYPSDISVEWEKNGKAEDNYKTTPAVLDSDGSYFLYSKLSVPTS EWQRGDVFTCSVMHEALHNHYTQKSISRSPGK |
| 66 | MGNVFFLLLFSLTHFPLAQQSRCTLTVGISSYHSSPCSPTQPVCTWNLDLN SLTTDQRLHPPCPNLITYSGFHKTYSLYLFPHWIKKPNRQGLGYYSPSYND PCSLQCPYLGCQSWTCPYTGPVSSPSWKFHSDVNFTQEVSQVSLRLHFSK CGSSMTLLVDAPGYDPLWFITSEPTQGSVDVPRDCGCKPCICTVPEVSSVF IFPPKPKDVLTITLTPKVTCVVVDISKDDPEVQFSWFVDDVEVHTAQTQPR EEQFNSTFRSVSELPIMHQDWLNGKEFKCRVNSAAFPAPIEKTISKTKGRP KAPQVYTIPPPKEQMAKDKVSLTCMITDFFPEDITVEWQWNGQPAENYK NTQPIMDTDGSYFVYSKLNVQKSNWEAGNTFTCSVLHEGLHNHHTEKSL SHSPGK |
| 67 | SRCTLTVGISSYHSSPCSPTQPVCTWNLDLNSLTTDQRLHPPCPNLITYSGF HKTYSLYLFPHWIKKPNRQGLGYYSPSYNDPCSLQCPYLGCQSWTCPYTG PVSSPSWKFHSDVNFTQEVSQVSLRLHFSKCGSSMTLLVDAPGYDPLWFI TSEPTQGSVDVPRDCGCKPCICTVPEVSSVFIFPPKPKDVLTITLTPKVTCV VVDISKDDPEVQFSWFVDDVEVHTAQTQPREEQFNSTFRSVSELPIMHQD WLNGKEFKCRVNSAAFPAPIEKTISKTKGRPKAPQVYTIPPPKEQMAKDK VSLTCMITDFFPEDITVEWQWNGQPAENYKNTQPIMDTDGSYFVYSKLNV QKSNWEAGNTFTCSVLHEGLHNHHTEKSLSHSPGK |
| 68 | MGNVFFLLLFSLTHFPLAQQSRCTLTVGISSYHSSPCSPTQPVCTWNLDLN SLTTDQRLHPPCPNLITYSGFHKTYSLYLFPHWIKKPNRQGLGYYSPSYND PCSLQCPYLGCQSWTCPYTGPVSSPSWKFHSDVNFTQEVSQVSLRLHFSK CGSSMTLLVDAPGYDPLWFITSEPTQGSVSKGEELFTGVVPILVELDGDVN GHKFSVSGEGEGDATYGKLTLKFICTTGKLPVPWPTLVTTLTYGVQCFSR YPDHMKQHDFFKSAMPEGYVQERTIFFKDDGNYKTRAEVKFEGDTLVNR IELKGIDFKEDGNILGHKLEYNYNSHNVYIMADKQKNGIKVNFKIRHNIED GSVQLADHYQQNTPIGDGPVLLPDNHYLSTQSALSKDPNEKRDHMVLLE FVTAAGITLGMDELYK |
| 69 | SRCTLTVGISSYHSSPCSPTQPVCTWNLDLNSLTTDQRLHPPCPNLITYSGF HKTYSLYLFPHWIKKPNRQGLGYYSPSYNDPCSLQCPYLGCQSWTCPYTG PVSSPSWKFHSDVNFTQEVSQVSLRLHFSKCGSSMTLLVDAPGYDPLWFI TSEPTQGSVSKGEELFTGVVPILVELDGDVNGHKFSVSGEGEGDATYGKL TLKFICTTGKLPVPWPTLVTTLTYGVQCFSRYPDHMKQHDFFKSAMPEGY VQERTIFFKDDGNYKTRAEVKFEGDTLVNRIELKGIDFKEDGNILGHKLEY NYNSHNVYIMADKQKNGIKVNFKIRHNIEDGSVQLADHYQQNTPIGDGP VLLPDNHYLSTQSALSKDPNEKRDHMVLLEFVTAAGITLGMDELYK |
| 70 | MVADPPRDSKGLAAAEPTANGGLALASIEDQGAAAGGYCGSRDQVRRCL RANLLVLLTVVAVVAGVALGLGVSGAGGALALGPERLSAFVFPGELLLR LLRMIILPLVVCSLIGGAASLDPGALGRLGAWALLFFLVTTLLASALGVGL ALALQPGAASAAINASVGAAGSAENAPSKEVLDSFLDLARNIFPSNLVSA AFRSYSTTYEERNITGTRVKVPVGQEVEGMNILGLVVFAIVFGVALRKLG PEGELLIRFFNSFNEATMVLVSWIMWYAPVGIMFLVAGKIVEMEDVGLLF ARLGKYILCCLLGHAIHGLLVLPLIYFLFTRKNPYRFLWGIVTPLATAFGTS SSSATLPLMMKCVEENNGVAKHISRFILPIGATVNMDGAALFQCVAAVFI AQLSQQSLDFVKIITILVTATASSVGAAGIPAGGVLTLAIILEAVNLPVDHIS LILAVDWLVDRSCTVLNVEGDALGAGLLQNYVDRTESRSTEPELIQVKSE LPLDPLPVPTEEGNPLLKHYRGPAGDATVASEKESVM |
| 71 | |

### BRIEF DESCRIPTION OF THE DRAWINGS

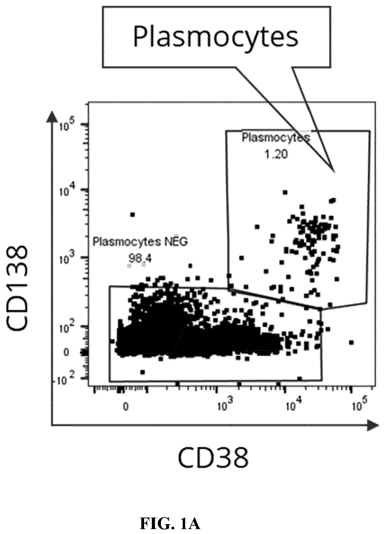

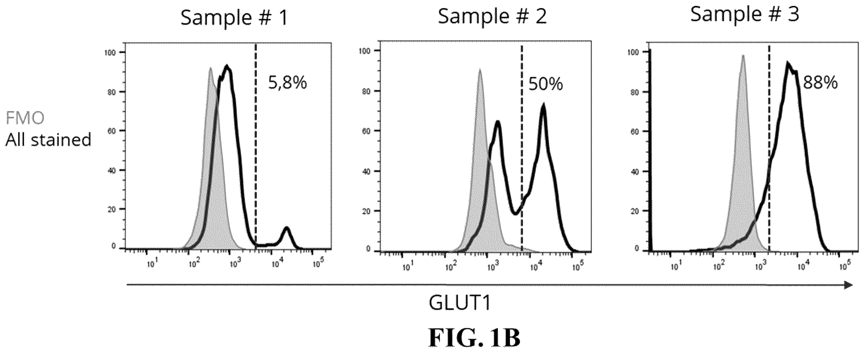

**Figure 1** is a combination of plots and graphs demonstrating identification of plasma cells and the expression of GLUT1 on their cell surface. **Figure 1A** depicts a representative flow cytometry dot plot to identify plasma cells by selecting cells that co-expressed CD38 and CD138. **Figure 1B** shows the heterogeneity observed in GLUT1 expression on plasma cells from bone marrow of patients with multiple myeloma.

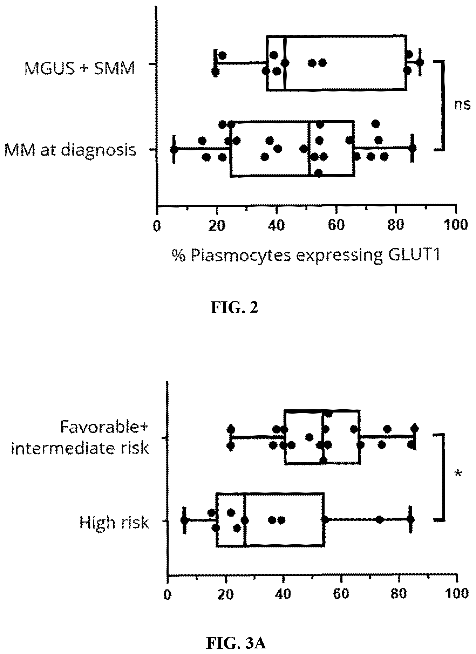

**Figure 2** is a graph showing GLUT1 expression on plasma cells at distinct stages of multiple myeloma. In particular, it shows the distribution of proportion of plasmocytes expressing GLUT1 from bone marrow of 35 patients at different stages of multiple myeloma in a cross-sectional study. Early stages MGUS and SMM are grouped together, distinct from newly diagnosed MM. Mann-Whitney test was performed to analyze the statistical difference between the groups. MGUS = monoclonal gammopathy of Unkown Significance ; SMM = Smoldering Multiple Myeloma ; MM = Multiple Myeloma.

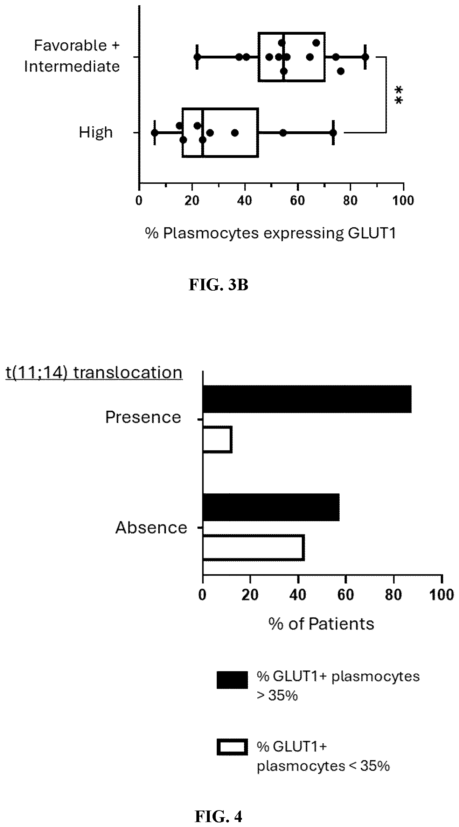

**Figure 3** is a combination of graphs depicting the association of GLUT1 expression on plasma cells with prognostic risk factor in Myeloma patients. **Figure 3A****:** patients (n=35) with MGUS, SMM and MM at diagnosis were categorized into 2 groups based on their prognostic risk factor as defined by the IMWG consensus on risk stratification in multiple myeloma. The figure shows comparison of proportion of plasma cells expressing GLUT1 between patients with favorable to intermediate versus high prognostic risk factor. Mann-Whitney test was performed to analyze the statistical difference between groups. **Figure 3B****:** Patients (n=22) with MM at diagnosis were categorized into 2 groups based on their prognostic risk factor as defined by the clinicians. It shows the distribution and comparison of GLUT1 expression on plasma cells between patients with favorable to intermediate versus high prognostic risk factor. Mann-Whitney test was performed to analyse the statistical difference.
**Figure 4** is a histogram showing the association of GLUT1 expression on plasma cells with t(11;14) chromosomal translocation in patients with MM at diagnosis. In particular, patients (n=22) with MM at diagnosis were divided into 2 groups based on the presence or absence of t(11;14) chromosomal translocation. The black bar depicts the proportion of patients in each group with GLUT1+ plasma cells above 35% whereas the white bar signifies below 35%.

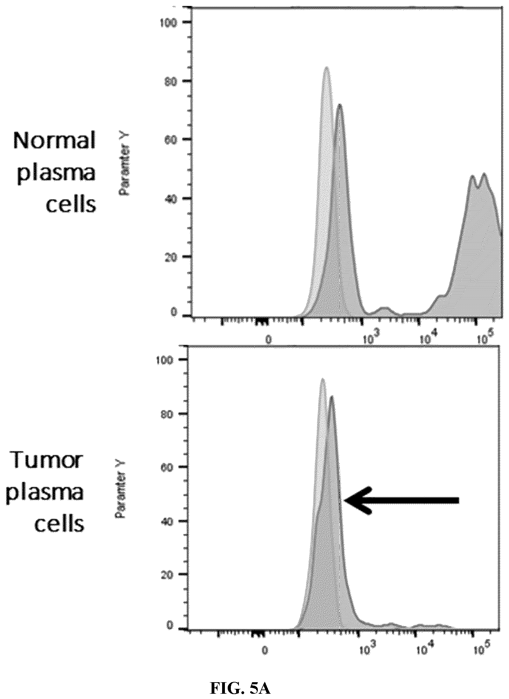

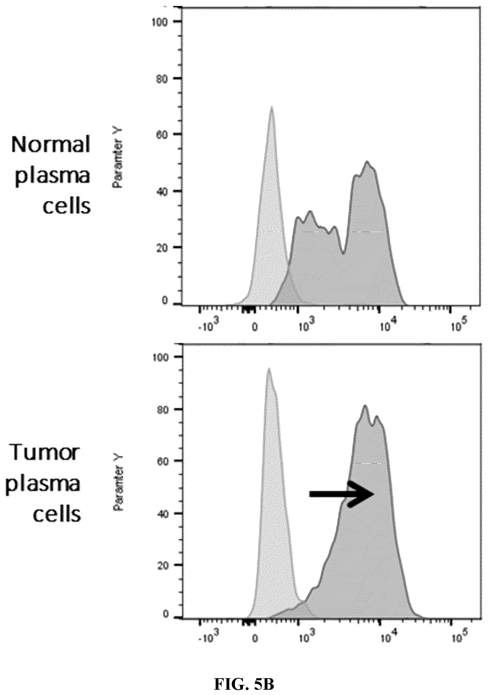

**Figure 5** is a combination of plots showing the expression of GLUT 1 and ASCT2 on normal and abnormal plasmocytes. **Figure 5A****:** Illustrative flow cytometry dot plot showing expression of GLUT1 on plasmocytes from a multiple myeloma patient. In dark grey are labelings obtained with RBDs against GLUT1, whereas the light grey represents background noise observed in the channels where signals from RBDs are detected. **Figure 5B****:** Illustrative flow cytometry dot plot showing expression of ASCT2 on plasmocytes from a multiple myeloma patient. In dark grey are labelings obtained with RBDs against ASCT2, whereas the light grey represents background noise observed in the channels where signals from RBDs are detected.

### EXAMPLES

The present invention is further illustrated by the following examples.

### Example 1: Plasma cells from bone marrow of multiple myeloma subjects express distinct level of GLUT 1

### Materials and Methods

### Measurement of GLUT1 on the surface of bone marrow plasmocytes by flow cytometry

50µL bone marrow sample was labelled with 50µL mix of RBDs against GLUT1 and ASCT2 along with antibodies against CD38, CD138, CD45, CD56, CD19 & viability marker at room temperature for 20 minutes. Cells were then washed with Dulbecco's phosphate buffered saline (DPBS) and fixed, followed by another round of DPBS wash and subsequent acquisition on ZE5 Cell Analyzer (4 lasers with 405, 488, 561 and 640 nm excitation; Bio-Rad). Analysis was performed using FlowJo software (BD biosciences). Debris, doublets and dead cells were excluded from the analysis. Plasma cells were identified as CD138+ CD38+ cells. Fluorescence minus one (FMO) control was used to establish background levels in RBD channels (Peric, Barragan et al. 2015).

### Classification of subjects

Subjects with a monoclonal gammopathy were classified as MGUS, SMM, MM at diagnosis and Relapse based on Mayo Clinic criteria (Rajkumar, Am J Hematol. 2024 Jun 28).

### Cytogenetic abnormalities

All included patients had cytogenetic analysis performed by karyotype analysis and or by FISH.

### Results

### Level of GLUT1 expression on plasma cell derived from bone marrow of Multiple Myeloma patients

Multiple myeloma is a malignancy of terminally differentiated plasma cells, and patients typically present with bone marrow infiltration of clonal plasma cells and monoclonal protein in the serum and/or urine. Distinguishing symptomatic multiple myeloma that requires treatment from the precursor stages of monoclonal gammopathy of undetermined significance (MGUS) and smoldering multiple myeloma (SMM) is important, as observation is the standard for those conditions. Overall, the prognostic factors are not fully understood, and more biomarkers are required to refine the prognosis and make the best medical decision.

We hypothesize that metabolic reprogramming is a key step in the pathophysiology of monoclonal gammopathy. To this end, we measured glucose transporter (Glut1) and glutamine transporter (ASCT2) on the surface of bone marrow plasma cells by FACS.

After acquisition of labeled bone marrow on ZE5 cytometer, samples were analyzed by FlowJo. Debris, doublets and dead cells were excluded from analysis and plasma cells were identified as cells that co-expressed CD38 and CD138 (Figure 1A). These cells were then assessed for their surface expression of GLUT1. FMO was used as a control to set-up GLUT1 positivity threshold.

GLUT1 expression was heterogeneous across multiple myeloma patients ranging from 5% to around 88% (Figure 1B).

### Example 2: Level of GLUT1 expression on plasma cells at distinct stages of multiple myeloma

### Materials and Methods

### See the example 1

### Results

Since multiple myeloma is a very heterogenous disease, we investigated GLUT1 expression on the surface of plasma cells across different stages of multiple myeloma in a cross-sectional study. Bone marrow from 35 patients were analysed. Early stages of the disease namely MGUS and SMM were grouped together, which are distinct from newly diagnosed MM (Figure 2). Mann-Whitney test was performed to analyze the statistical difference between the groups. This difference is not significant.

### Example 3: Risk factors and expression of GLUT1 on plasma cells derived from bone marrow of patients with MGUS, SMM and Multiple Myeloma at diagnosis

### Materials and Methods

### See the example 1

### Risk factors

Risk was determined using Crab criteria and genetic anomalies as low, intermediate and high risks (Chng et al., Leukemia. 2014 Feb;28(2):269-77).

### Results

Thirty-five patients with MGUS, SMM and MM at diagnosis were categorized into 2 groups based on their prognostic risk factor as defined by the IMWG consensus on risk stratification in multiple myeloma. Patients with favorable to intermediate risk were compared with those having high risk factor for their expression of GLUT1 on plasma cells. Mann-Whitney test was performed to analyze the statistical difference between the groups. A high percentage of plasma cells expressed GLUT1 on their cell surface in patients with favorable and intermediate risk. In contrast, high-risk patients exhibited significantly lower levels of GLUT1 (Figure 3A).

Of note, higher levels of GLUT1 were also observed in patients with MM at diagnosis with favorable and intermediate risk, as compared with patients with MM at diagnosis with high risk (Figure 3B).

Altogether, these data indicate a relationship between proportion of GLUT1 positive plasma cells and multifactorial risk stratification in patients with MGUS, SMM and MM.

### Example 4: Association of GLUT1 expression on plasma cells with t(11;14) chromosomal translocation in patients with MM at diagnosis

### Materials and Methods

### See the example 1

### Results

Multiple myeloma harboring t(11;14) represents a unique disease subset as t(11;14)-positive myeloma cells exhibit biological features that are distinct from t(11;14)-negative myeloma cells, including overexpression of cyclin D1, higher levels of the antiapoptotic protein BCL-2 which explains their sensitivity to agents targeting the BCL2 family.

GLUT1 expression was analyzed on plasma cells surface in patients with multiple myeloma at diagnosis with (t(11;14)+) or without (t(11;14)-) chromosomal translocation. (Figure 4).

The group with t(11;14) translocation comprised of 87.5% patients with more than 35% of GLUT1 expressing plasma cells.

T(11;14) is known as being an efficacy marker of Bcl2 inhibitors such as venetoclax. Thus, more than 35% of GLUT1 positive plasmocytes is a marker of sensitivity to Bcl2 inhibitors.

### Example 5: Expression of GLUT1 & ASCT2 on plasma cells

### Materials and Methods

### See the example 1

### Results

High level of GLUT1 expression signifies cells undergoing glycolysis. However, as cell metabolism dynamics is not governed by just one pathway, we assessed the cell surface expression of ASCT2 which is involved in both oxidative phosphorylation as well as glutaminolysis. ASCT2 expression was observed on the surface of plasma cells derived from bone marrow of subjects with multiple myeloma.

Illustrative flow cytometry dot plot showing expression of GLUT1 and ASCT2 on plasma cells from multiple myeloma subjects (Figures 5A and 5B). In dark grey are labelings obtained with RBDs against GLUT1 and ASCT2, whereas the light grey represents background noise observed in the channels where signals from RBDs are detected.

When GLUT1 is downregulated by plasma cells, ASCT2 is overexpressed. Quantifying both nutrient transporters on plasma cells allows for robust characterization of the cell metabolic reprogramming, which is a hallmark of bad prognosis (as per Figures 3 and 4).

## Claims

1. A method for stratifying prognosis of subjects suffering from a hematologic disorder, comprising a step of determining the proportion of plasma cells, lymphocytes or blasts expressing GLUT1 among total plasma cells, lymphocytes or blasts in a sample obtained from the subjects.

2. The method according to claim **1,** wherein the hematologic disorder is monoclonal gammopathy of undetermined significance (MGUS), plasmacytoma, smoldering MM (SMM) or multiple myeloma (MM), preferably wherein the hematologic disorder is MGUS, SMM or MM.

3. The method according to any one of claims **1** to **2,** wherein the method is for stratifying, in subjects suffering from MGUS, plasmacytoma or SMM, the risk of progression to MM, and preferably wherein the method is for stratifying, in subjects suffering from MGUS or SMM, the risk of progression to MM.

4. The method according to any one of claims **1** to **2,** wherein the method is for stratifying prognosis, and in particular life expectancy, of subjects suffering from MM.

5. The method according to any one of claims **1** to **4,** wherein said method comprises the steps of:
a) determining the proportion of plasma cells expressing GLUT1 among total plasma cells in samples obtained from the subjects,
b) comparing the proportion of plasma cells expressing GLUT1 determined in step a) with a reference value, and
c) stratifying subjects in risk groups or prognosis groups based on the correlation of the proportion of plasma cells expressing GLUT1 in step a) with the reference value.

6. The method according to claim **5,** wherein the reference value is either:
• a personalized reference, determined earlier in a same subject, or
• a proportion of plasma cells expressing GLUT1 determined in a reference population.

7. The method according to any one of claims **5** to **6,** wherein a subject is assigned in a high-risk group or poor prognosis group when the proportion of plasma cells expressing GLUT1 measured in step a) is lower than the reference value.

8. A method for determining whether a subject suffering from a hematologic disorder will achieve or is achieving a response with modulator of the BCL-2 family proteins, comprising a step of determining the proportion of plasma cells, lymphocytes or blasts expressing GLUT1 among total plasma cells, lymphocytes or blasts in a sample obtained from the subject.

9. The method according to claim **8,** wherein said method comprises the steps of:
a) determining the proportion of plasma cells, lymphocytes or blasts expressing GLUT1 among total plasma cells, lymphocytes or blasts in a sample obtained from the subject,
b) comparing the proportion of plasma cells, lymphocytes or blasts expressing GLUT1 determined in step a) with a reference value, and
c) concluding that the subj ect will achieve or is achieving a response with the modulator based on the correlation of the proportion of plasma cells, lymphocytes or blasts expressing GLUT1 in step a) with the reference value.

10. The method according to claim **9,** wherein it is concluded that the subject will achieve or is achieving a response with the modulator when the proportion of plasma cells, lymphocytes or blasts expressing GLUT1 measured in step a) is higher than the reference value.

11. The method according to any one of claims **8** to **10,** wherein the hematologic disorder is selected from the group comprising or consisting of leukemia, lymphoma, MM, plasmacytoma, MGUS, SMM, myelodysplastic syndromes (MDS) and myeloproliferative neoplasms (MPNs), preferably wherein the hematologic disorder is MM.

12. The method according to any one of claims **8** to **11,** wherein the method is for determining whether a subject suffering from a hematologic disorder will achieve or is achieving a response with an inhibitor of anti-apoptotic BCL-2 family proteins, preferably wherein said inhibitor of anti-apoptotic BCL-2 family proteins is selected from the group comprising or consisting of venetoclax (ABT-199), oblimersen (G3139), obatoclax (GX15-070), navitoclax (ABT-263), pelcitoclax (APG-1252), R-(-)-gossypol acetic acid (AT-101), LP-118, ABT-737, S55746 (BLC201), APG-2575 (lisaftoclax), AZD4320, AZD0466, BM-1197, S44563, DC-B01, A1210477, S63845, DT2216, 753b and WH244, more preferably wherein said inhibitor of anti-apoptotic BCL-2 family proteins is venetoclax.

13. The method according to any one of claims **1** to **12,** wherein the method further comprises a step of measuring the expression of ASCT2 by plasma cells, lymphocytes or blasts in the sample obtained from the subjects.

14. The method according to any one of claims **1** to **13,** wherein GLUT1 and/or ASCT2 expression levels are assessed at the protein level, preferably the measurement of the expression levels of GLUT1 and/or ASCT2 correspond to the detection and/or quantification of GLUT1 and/or ASCT2 on the cell surface, more preferably by detecting and/or quantifying binding of a ligand to GLUT1 or ASCT2, wherein preferably, said ligand is an antibody or is a receptor-binding domain ligand (RBD) comprising a part or the totality of a receptor-binding domain (RBD) derived from the soluble part of a glycoprotein of an enveloped virus.

15. A kit comprising means for detecting GLUT1, and optionally ASCT2, preferably wherein said kit is for implementing a method according to any one of claims **1** to **14.**
